(19) **Europäisches Patentamt**

**European Patent Office**

**Office européen des brevets**

(11) **EP 1 504 766 A1**

(12) **EUROPEAN PATENT APPLICATION**
published in accordance with Art. 158(3) EPC

(43) Date of publication:
**09.02.2005 Bulletin 2005/06**

(51) Int Cl.[7]: **A61K 47/48**, A61K 35/76, A61K 48/00

(21) Application number: **03728015.3**

(22) Date of filing: **30.04.2003**

(86) International application number:
**PCT/JP2003/005527**

(87) International publication number:
**WO 2003/092738 (13.11.2003 Gazette 2003/46)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **30.04.2002 JP 2002129026**

(71) Applicant: **Dnavec Research Inc.**
**Tsukuba-shi, Ibaraki 305-0856 (JP)**

(72) Inventors:
• **SAKAKIBARA, Hiroyuki, Dnavec Research Inc.**
**Tsukuba-shi, Ibaraki 305-0856 (JP)**

• **HARA, Hiroto, c/o Dnavec Research Inc.**
**Tsukuba-shi, Ibaraki 305-0856 (JP)**
• **UEDA, Yasuji, c/o Dnavec Research Inc.**
**Tsukuba-shi, Ibaraki 305-0856 (JP)**
• **HASEGAWA, Mamoru, c/o Dnavec Research Inc.**
**Tsukuba-shi, Ibaraki 305-0856 (JP)**
• **YOU, Jun, c/o Dnavec Research Icn.**
**Tsukuba-shi, Ibaraki 305-0856 (JP)**

(74) Representative: **VOSSIUS & PARTNER**
**Siebertstrasse 4**
**81675 München (DE)**

(54) **DRUG- OR GENE-CARRIER COMPOSITION HAVING LOWERED HEMAGGLUTININ ACTIVITY**

(57) The present invention provides pharmaceutical- or gene-carrier compositions with reduced hemagglutinating activity. By attaching a compound to a minus-strand RNA virus envelope protein having hemagglutinating activity, a pharmaceutical- or gene-carrier composition with lower hemagglutinating activity than a composition to which the compound has not been attached can be successfully constructed. For example, an embodiment of the present invention provides a viral vector whose erythrocyte agglutination activity and hemolytic activity are significantly lowered, and whose stability in blood is remarkably elevated. The pharmaceutical- or gene-carrier compositions provided in this invention can be preferably used for transferring pharmaceuticals or genes *in vivo*.

**EP 1 504 766 A1**

**Description**

Technical Field

The present invention relates to carriers for delivering pharmaceuticals or genes into cells, whose hemagglutinating activity is reduced by modifying an envelope protein of a minus-strand RNA virus.

Background Art

The Sendai virus (SeV) contains two types of envelope proteins: hemagglutinin-neuraminidase (HN) and fusion protein (F). HN binds to sialic acid on cell surface and followed by fusion mediated by F protein, allowing a direct, rapid, and highly efficient infection of SeV into cytoplasm. Furthermore, since sialic acid is present on almost all cell surfaces, SeV can infect a wide range of cells. In order to apply these kinds of extremely infective SeV to gene therapy, a recombinant SeV vector carrying various genes has been developed (Shiotani et al., Gene Therapy, 2001, 8, 1043-1050), and research is also progressing on a drug delivery system where genes and various pharmaceuticals are encapsulated in the aqueous core of liposomes which comprise SeV envelope proteins (Ramani et al., FEBS Letters, 1997, 404, 164-168; Isaka et al., Experimental Nephrology, 1998, 6, 144-147).

SeV vectors have many such advantages, however, their broad infectivity can also be a disadvantage. SeV vectors also fuse with erythrocytes which contain a large amount of sialic acid, triggering a high hemolytic activity. Thus, improvement is desired from the safety point of view. Furthermore, SeV vector fusion with erythrocytes means that the vector cannot deliver the required amount of pharmaceuticals to target cells, suggesting that SeV vector may be extremely unstable in blood. Therefore, methods for administering SeV vectors *in vivo* are predicted to be confined to methods that do not mediate blood, such as temporarily stopping blood flow or perfusion methods, or to local administration methods for sites that are little influenced by blood components.

In order to resolve these safety- and stability-associated problems, the hemagglutinating activity of HN protein must be eliminated or decreased to reduce its binding with erythrocytes. However, eliminating hemagglutinating activity leads to reduced SeV binding to the target cells to be introduced with pharmaceuticals, which can be readily inferred to significantly reduce the efficiency of drug delivery. Therefore, some ingenuity is required.

So far, there have been many reports of studies that eliminated hemagglutinating activity by specifically reducing the SeV HN protein with dithiothreitol (DTT). However, most of these studies have used hemolytic activity toward erythrocytes as an indicator. For example, Tomasi et al., in FEBS Letters, 1982, 143 (2), 252-256, reported that reducing SeV with DTT eliminates its hemagglutinating activity. However, they merely report that newly introducing erythrocyte-recognizing antibody molecules into SH groups (formed by DTT reduction) in HN protein molecules, complements the ability of SeV to bind to erythrocytes, exhibiting a revived hemolytic activity. Furthermore, since fusion activity is greatly reduced, doubts remain as to the possibilities of this method. Gitman et al., in Biochemistry, 1985, 24, 2762-2768, reported the chemical modification of SeV envelope proteins. They succeeded in eliminating hemagglutinating activity, but at the same time, they also eliminated fusion ability completely. They report that to confer fusion ability, an unmodified envelope protein must be newly incorporated, which leads to a revival of hemagglutinating activity.

Several studies that target cells have been also reported. For example, Bagai et al., in Journal of Virology, 1993, 67 (6), 3312-3318, proposed a drug delivery system for the sugar chain-specific infection of F protein, which comprises the steps of: solubilizing SeVs whose HN has been reduced with surfactants; and reconstituting virions consisting only of F proteins. Ramani et al., in FEBS Letters, 1997, 404, 164-168, also reported the results of *in vitro* gene introduction using a similar system. Whilst these methods can indeed eliminate hemagglutinating activity and fuse SeV only with target cells, some problems remained to be solved. For example, F protein is also considerably reduced along with HN protein. Furthermore, not all of the envelope proteins are incorporated into virions in the reconstitution process (Ponimaskin et al., Virology, 2000, 269, 391-403), thus impairing the fusion ability of the reconstituted carriers and resulting in an increased dosage. Also, in the preparation process, the requirement of a large amount of virus, troublesome removal of surfactant, heterogeneity of the reconstituted virions, and such provide ample room for improvement.

Thus, hitherto there has been no known drug delivery system based on an envelope protein of a minus-strand RNA virus that has significantly reduced hemagglutinating activity but retains the ability to deliver drugs into cells.

Disclosure of the Invention

The objective of the present invention is to provide a carrier, in which only hemagglutinating activity is significantly reduced, and which retains the ability to introduce a pharmaceutical or gene into cells, by modifying an envelope protein in a minus-strand RNA virus.

In the development of pharmaceuticals and drug formulations, Drug Delivery System (DDS) is an important technique as a methodology to reduce undesirable effects and enable intended functions. The present inventors thought that chemically modifying a minus-strand RNA viral envelope protein, which is one DDS technique, can reduce activity against erythrocytes and retain the ability for introduction into cells. For this, it is preferable to selectively modify the envelope protein responsible for the hemagglutinating activity, to reduce the activity while minimizing, as much as possible, the effect on the envelope protein critical to fusion.

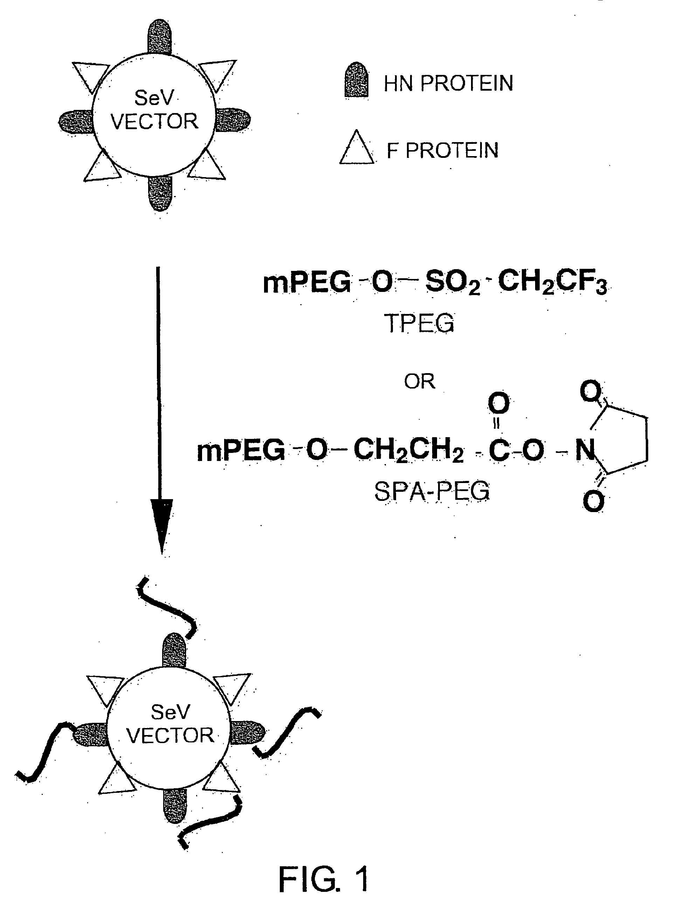

The present inventors carried out exhaustive studies and discovered that modification with polymers such as polyethyleneglycol (PEG) effectively reduces hemagglutination. For example, in a PEG-modified SeV vector, obtained by covalently binding activated PEG to an amino group in the envelope protein of Sendai virus (SeV) (Fig. 1), a markedly reduced hemagglutination response (HAU) was apparent for the amount of PEG reacted. Although there was a concern that vector infectivity of target cells might also decrease along with the reduction of HAU, the degree of reduction in the ability of the modified vector to introduce genes into cells was small compared to that of HAU, confirming that sufficient vector infectivity was retained (Tables 1 and 2). In this case, it was revealed that a larger molecular weight of PEG can enhance the ability to introduce genes into cells. Furthermore, the method for preparing PEG-modified vector were very rapid and easy.

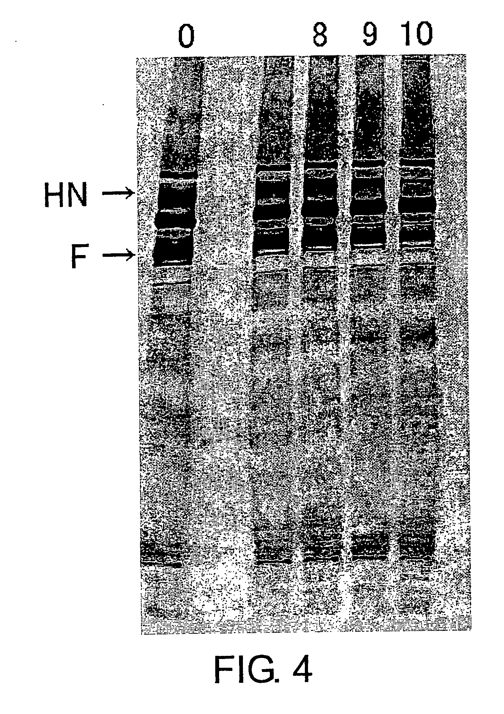

In the electrophoretic patterns of the constituent proteins of PEG-modified SeV vector, HN proteins drastically decreased and instead, new macromolecules were observed to appear. On the other hand, the decrease in F proteins was relatively small, suggesting that PEG preferentially modifies HN protein rather than binding to F protein. This was thus inferred to be a contributing factor in preserving fusion ability while drastically reducing HAU (Figs. 4, 15, and 16).

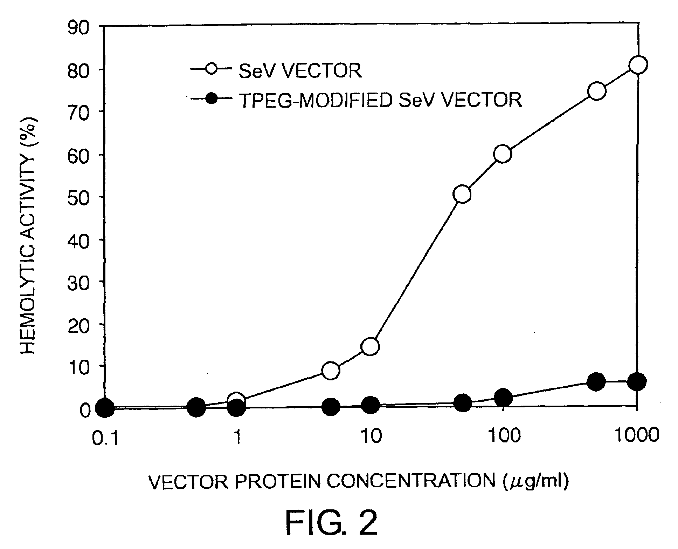

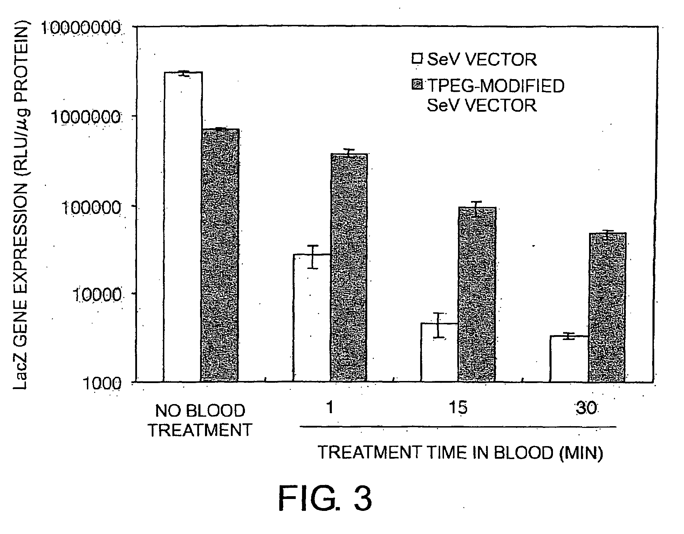

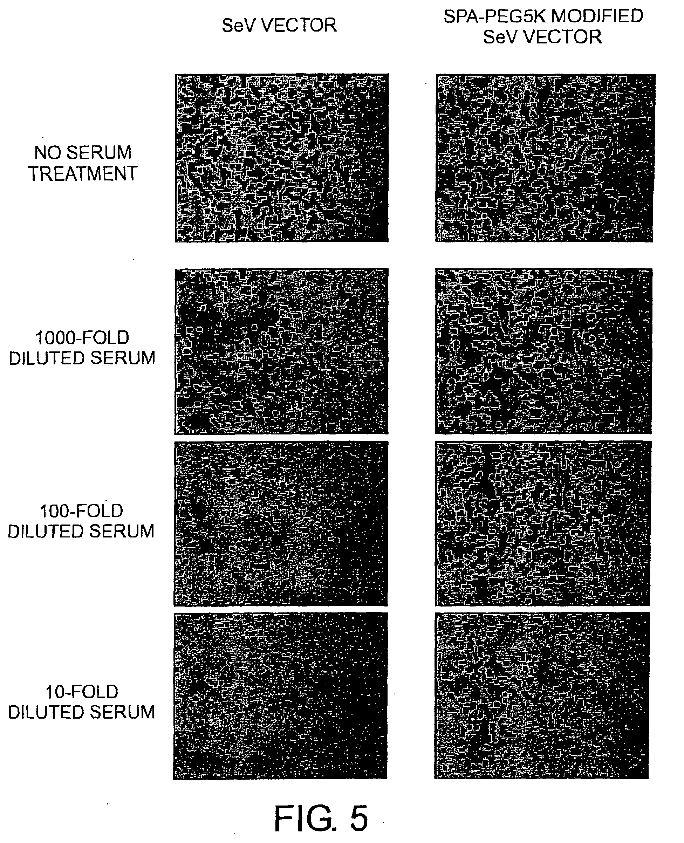

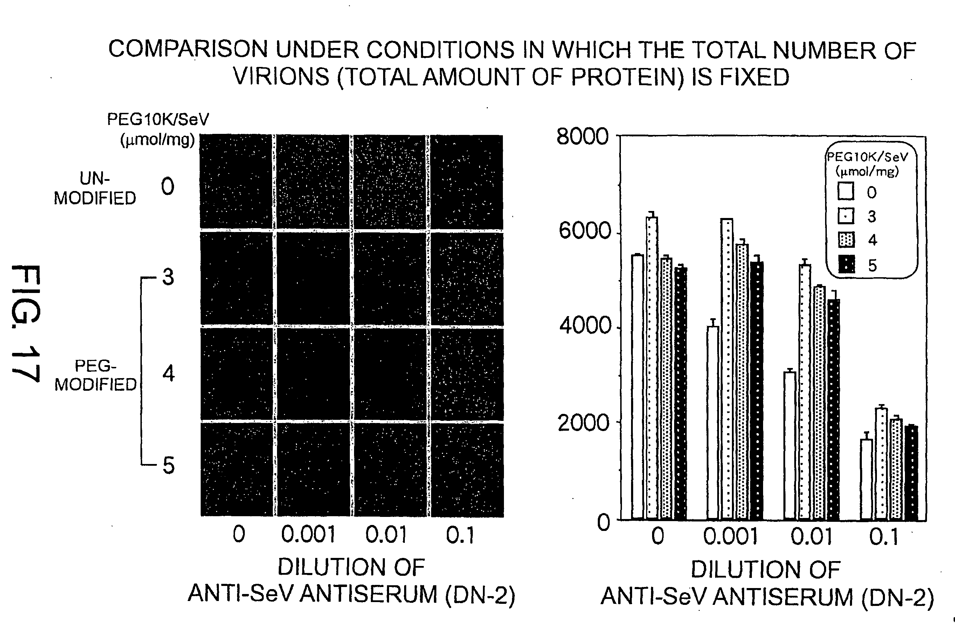

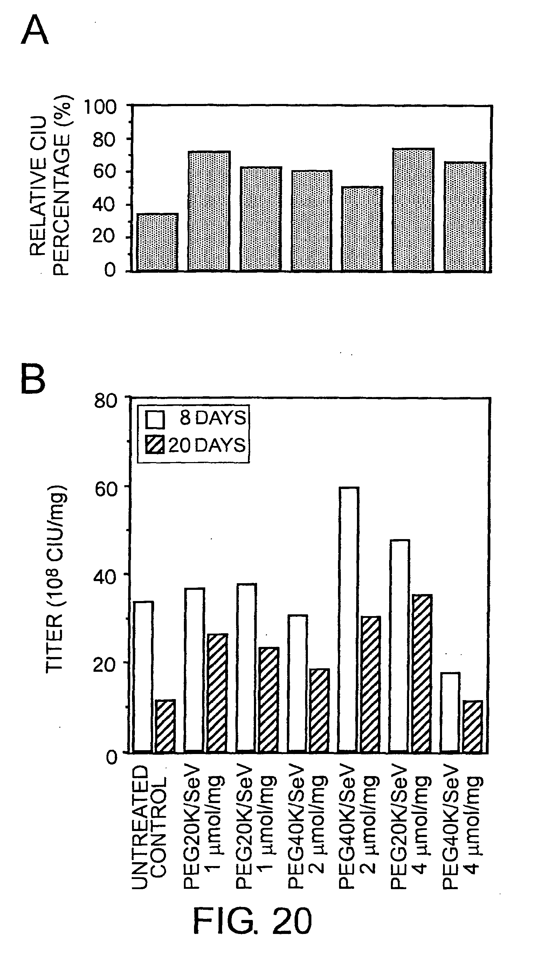

The hemolytic activity of PEG-modified SeV vector toward rat blood was significantly reduced as a reflection of reduced HAU, indicating this vector to be highly excellent from the safety point of view (Fig. 2). Furthermore, although the gene-introducing ability of the SeV vector was markedly reduced upon contact with the blood, the modified vector retained a sufficient level of gene-introducing ability even after the blood treatment (Figs. 3 and 20). These results indicate that modification not only has the effect of reducing binding to erythrocytes, but also, at the same time, can impart a stabilizing effect on factors such as complements in blood, that are likely to destabilize vector activity. Furthermore, since the PEG-modified vector could actually introduce genes stably, even in the presence of a neutralizing antibody (Figs. 5 and 17), PEG modification was thought to impart stabilizing effects on various components of living body.

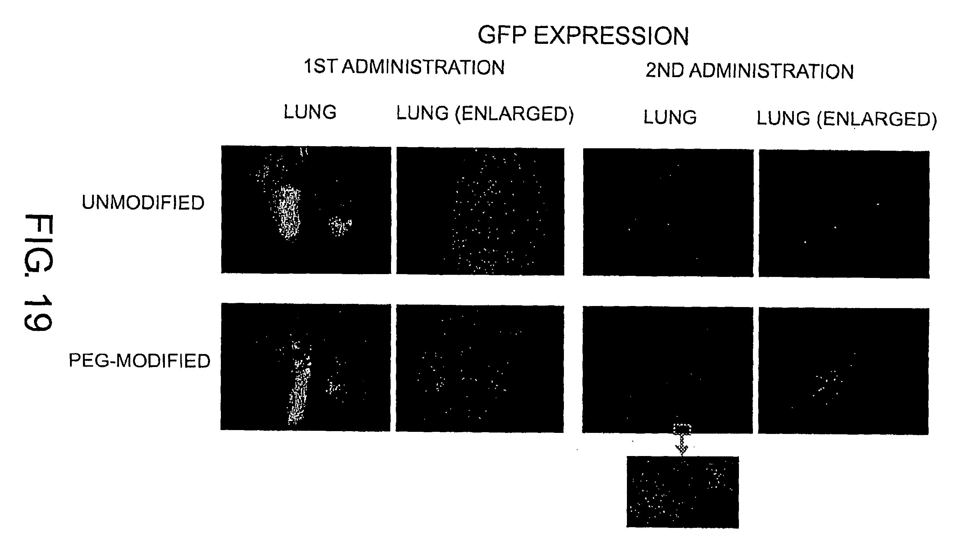

In addition, on examining the effect of PEG-modified vector administrated in vivo, the gene-introducing efficiency of this vector in multiple administrations was clearly higher than that of the unmodified vector, confirming that the PEG-modified vector has a high gene-introducing ability *in vivo* (Fig. 19).

As described above, it was found that appropriately selecting conditions of PEG modification could reduce the interaction of SeV vector with erythrocytes and yet retain a high level of gene-introducing ability into cells.

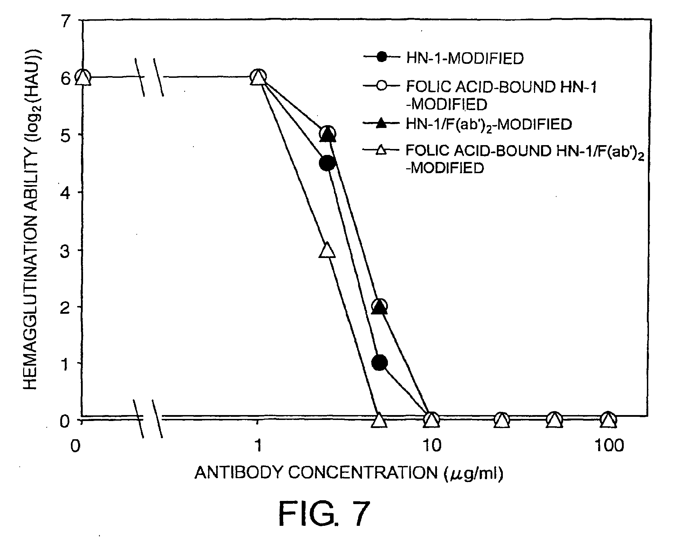

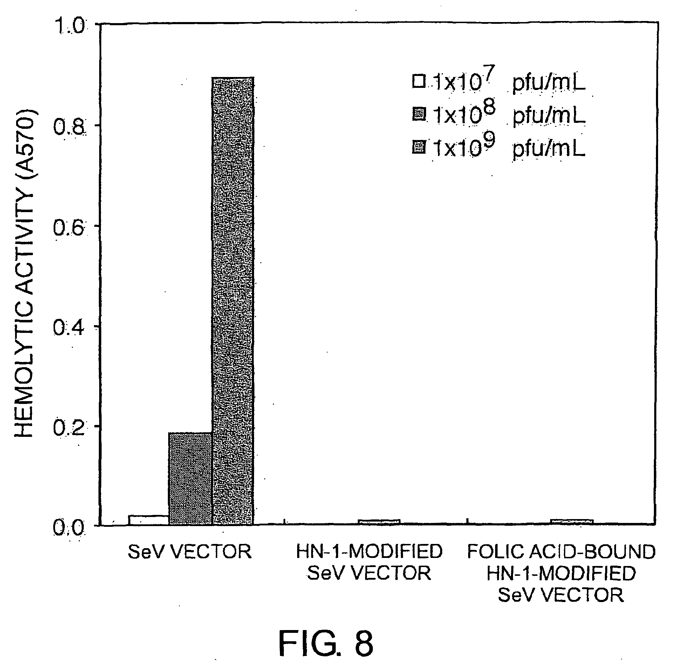

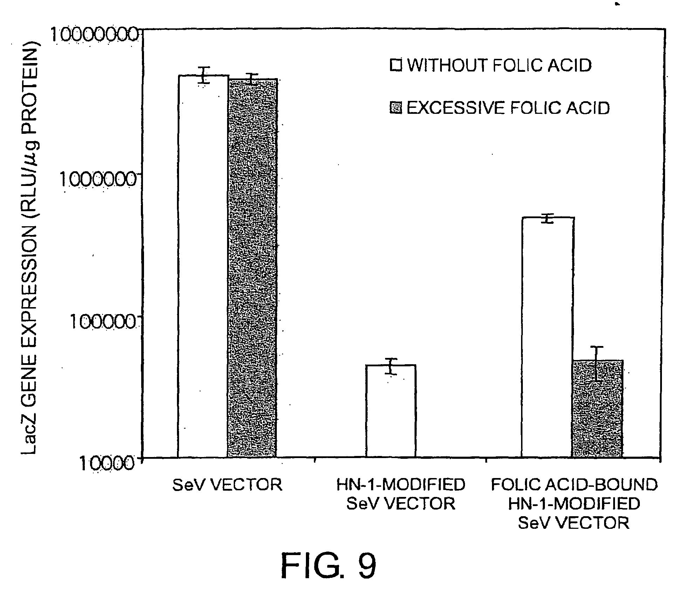

Furthermore, the present inventors also examined the use of a purified anti-HN protein monoclonal antibody (MAb), which selectively binds to envelope proteins responsible for hemagglutinating activities and reduces such activities (Miura et al., Exp. Cell Res., 1982, 141, 409-420). Sendai viral vectors treated with MAb showed no hemagglutination at all. Their hemagglutinating activity was completely blocked, and gene introduction into cells by the treated vector was not observed. When a target cell-binding ligand molecule was newly bound to the MAb molecule, Sendai viruses treated with this ligand-bound MAb (Fig. 5) showed neither hemagglutination nor hemolytic activity (Figs. 7, 10, and 11), and in spite of this, said treated viruses could introduce genes into potentially ligand-bound target cells with high efficiency (Figs. 8 and 9). Genes could not be introduced other than into target cells, and gene-introducing ability was eliminated in the presence of excess amounts of competitive substances (Table 4). These results prove that highly efficient gene introduction, exclusively into target cells, is possible despite the complete loss of the modified vector's hemagglutinating activity.

It is thought that this system may become applicable to introduction into various cells, by changing the ligand molecule, which is to be bound, into a molecule that responds to a receptor highly expressed on a target cell.

Furthermore, since this system can be readily prepared by mixing components just prior to use, it is very easy to use.

Such techniques can be also applied to virion formulations reconstituted from the constituent proteins of SeV envelope. In this case, it may not be necessary to reduce HN proteins as described above. If this technique is applied after solubilizing SeV without prior reduction and reconstituting virions that encapsulate required pharmaceuticals, carriers with greater fusion ability are formed with no effects on F protein due to reduction.

The present invention relates to pharmaceutical- or gene-carrier compositions whose hemagglutinating activity is reduced. More specifically, the present invention provides:

[1] a pharmaceutical- or gene-carrier composition which comprises a cell membrane comprising a minus-strand RNA virus envelope protein with hemagglutinating activity, wherein:

(a) a compound is added to the protein;

(b) the hemagglutinating activity is reduced compared to a composition to which the compound is not added; and

(c) the composition comprises the ability to introduce a pharmaceutical or gene into a target cell;

[2] the composition of [1], further wherein

(d) hemolytic activity toward erythrocytes is reduced compared to a composition to which the compound is not added;

[3] the composition of [1] or [2], which comprises an infective virion of the minus-strand RNA virus;

[4] the composition of [1] or [2], which comprises an inactivated minus-strand RNA virus, or an envelope portion of the virus;

[5] the composition of any one of [1] to [4], wherein the minus-strand RNA virus is a *Paramyxoviridae* family virus;

[6] the composition of [5], wherein the *Paramyxoviridae* family virus is the Sendai virus;

[7] the composition of any one of [1] to [6], wherein the compound added to the protein has a molecular weight of 1,800 or more;

[8] the composition of [7], wherein the compound added to the protein has a molecular weight of 4,500 or more;

[9] the composition of [8], wherein the compound added to the protein has a molecular weight of 16,000 or more;

[10] the composition of any one of [1] to [9], wherein the compound added to the protein is polyethylene glycol;

[11] the composition of any one of [1] to [10], wherein the protein further forms a complex with polyethyleneimine;

[12] the composition of any one of [1] to [11], wherein the protein forms a complex with a cell-binding compound;

[13] the composition of [12], wherein the cell-binding compound is bound to the compound added to the protein;

[14] the composition of [12] or [13], wherein the compound added to the protein is an antibody that binds to the protein or fragment thereof;

[15] the composition of any one of [12] to [14], wherein the cell-binding compound is a ligand for a receptor on the cell surface;

[16] the composition of [15], wherein the ligand is folic acid;

[17] an antibody bound to a cell-binding compound, wherein the antibody binds to a minus-strand RNA virus envelope protein comprising hemagglutinating activity, or a fragment thereof;

[18] the antibody or fragment thereof of [17], which is F(ab')$_2$;

[19] the antibody or fragment thereof of [17] or [18], wherein the cell-binding compound is a ligand for a receptor on a cell surface;

[20] the antibody or fragment thereof of [19], wherein the ligand is folic acid; and

[21] a pharmaceutical- or gene-carrier kit which comprises:

(a) an antibody or fragment thereof which binds to a minus-strand RNA virus envelope protein comprising hemagglutinating activity, and to which a cell-binding compound is bound; and

(b) a pharmaceutical- or gene-carrier composition comprising the envelope protein.

The present invention provides pharmaceutical- or gene-carrier compositions which comprise a cell membrane containing a minus-strand RNA virus envelope protein with hemagglutinating activity, wherein: (a) a compound is attached to the protein; (b) the hemagglutinating activity is reduced compared to a composition to which the compound is not attached; and (c) the composition comprises the ability to introduce the pharmaceutical or gene into a target cell. The compositions of this invention are complexes that comprise a cell membrane containing a minus-strand RNA virus envelope protein having hemagglutinating activity. Such complexes may be, for example, infective virions, inactivated minus-strand RNA viruses, or complexes comprising the envelope components of disintegrated minus-strand RNA viruses, such as reconstituted liposomes.

Infective virions infect cells and thus deliver a pharmaceutical or gene encapsulated in the virus into those cells. In particular, by incorporating a foreign gene into a viral genome, the gene can be expressed from the viral genome in cells infected with the infective virions. The infective virus may be a replication-competent virus or a replication-deficient virus. The term "replication-competent virus" means that when a virus infects host cells, it is replicated within those host cells to produce infective virions.

Minus-strand RNA viruses can be reconstituted by transcribing viral genomic RNAs within cells, in the presence of viral proteins required for the transcription and replication of this genomic RNA. Recombinant minus-strand RNA viruses can be reconstituted using methods known in the art (WO 97/16539; WO 97/16538; WO 00/70055; WO 00/70070; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404; Hasan, M. K. et al., J. Gen. Virol. 78: 2813-2820, 1997; Kato, A. et al., 1997, EMBO J. 16: 578-587; Yu, D. et al., 1997, Genes Cells 2: 457-466). With these methods, minus-strand RNA viral vectors, including parainfluenza viruses, vesicular stomatitis viruses, rabies viruses, measles viruses, rinderpest viruses, Sendai viruses, and such, can be reconstituted from DNAs.

The recombinant viral vectors containing foreign genes can be obtained by inserting the foreign genes into the viral vector genomes. Any gene to be expressed in a target cell can be used as a foreign gene. The foreign genes may be genes encoding naturally-occurring proteins, or genes encoding proteins modified from naturally-occurring proteins by deletion, substitution, or insertion of nucleotide(s), so long as the gene encodes a protein functionally equivalent to the naturally-occurring protein. Alternatively, the foreign gene may encode a deletion derivative of a naturally-occurring protein or synthetic protein. For example, for gene therapy or such, viruses are reconstituted by inserting a therapeutic gene for a target disorder into a DNA which serves as the viral vector template (viral vector DNA), and then transcribing the resulting recombinant DNA. When introducing a foreign gene into a viral vector DNA, for example, into Sendai viral vector DNA, a sequence consisting of a multiple of six nucleotides is preferably inserted between, for example, the transcription ending (E) sequence and the transcription starting (S) sequence (Journal of Virology, Vol. 67, No. 8, 1993, p. 4822-4830). In paramyxovirus or such, a foreign gene can be inserted upstream and/ or downstream of the open reading frame (ORF) of the respective viral proteins (for example, ORFs of NP, P, M, F, HN, and L proteins). In order to avoid inhibiting the expression of genes upstream and downstream of the foreign gene, the E-I-S sequence (transcription ending sequence - intervening sequence - transcription starting sequence) is appropriately inserted upstream or downstream of the foreign gene, so that the E-I-S sequence units are arranged between respective genes.

The recovered viruses can be purified until substantially pure. Purification can be carried out by purification/ separation methods known in the art, including filtration, centrifugation, and column purification, or any combinations thereof. "Substantially pure" means that the virus accounts for the main proportion of a sample in which it exists as a component. Typically, a substantially pure viral vector can be identified by confirming that the proportion of the viral vector-derived protein is 50% or above of the total proteins comprised in the sample, preferably 70% or above, more preferably 80% or above, and further more preferabley 90% or above. Examples of specific methods for purifying minus-strand RNA viruses are those using cellulose sulfate esters or cross-linked polysaccharide sulfate esters (Examined Published Japanese Patent Applcations No. (JP-B) Sho 62-30752, JP-B No. 62-33879, and JP-B No. 62-30753), and those for adsorbing proteins to polysaccharides comprising fucose sulfate and/or degradation products thereof (WO 97/32010), and so on.

Inactivated viruses or reconstituted products from disintegrated viruses are used as transfection reagents for introducing a pharmaceutical or gene into cells. Minus-strand RNA viruses can be inactivated, for example, by UV irradiation. Furthermore, liposomes using inactivated viruses such as Hemaggulutinating Virus of Japan (HVJ)-liposome can be prepared by vibration and ultrasonication (see, "Liposome in Life Science / Laboratory Manual", Springer-Verlag Tokyo (1992), pp. 282-287). Furthermore, a composition (membrane fusion liposome) formed by mixing inactivated Sendai virions or liposomes and nucleic acids has been reported (Yasushi Kaneda: BIOTHERAPY, 8, 1265 (1994)). Alternatively, disintegrated viruses can be reconstituted by, for example, reconstituting liposomes from a solubilized virus to prepare so-called 'virosomes' (Bagai et al., (1993) Biochem. Biophys. Acta 1152, 15-25). Specifically, after solubilizing a minus-strand RNA virus with a surfactant such as Triton X-100, the insoluble RNP is removed by centrifugation. Virosomes can be prepared from reconstituted virions by removing the surfactant from the solubilized solution comprising the envelope and envelope proteins. Virosomes of uniform particle size can be collected by centrifugation (for example, at 12,000 rpm for ten minutes).

In a composition comprising infective virions, the infective virions may be either minus-strand RNA viruses or other viruses comprising a minus-strand RNA virus envelope protein with hemagglutinating activity. Pseudotyped viruses, reconstituted with the envelope protein of another virus, are well known in the art (WO 01/92508). A pseudotyped virus comprising an envelope protein of a minus-strand RNA virus can be manufactured, for example, by preparing a virosome or such that comprises an inactivated minus-strand RNA virus or an envelope protein of the virus; and fusing the virosome with another virus. Alternatively, a pseudotyped virus can be prepared by expressing in the packaging cell of another virus, an expression vector that expresses a minus-strand RNA virus envelope protein with hemagglutinating activity. Examples of viruses whose packaging cells are used to express the vector are retroviruses, specifically those belonging to the subfamily of oncoviruses comprising Moloney murine leukemia virus (MoMLV), murine stem cell virus (MSCV), and such (referred to as oncoviruses in this invention); and those belonging to the subfamily of lentiviruses, comprising human immunodeficiency virus (HIV) (e.g. HIV1 or HIV2), simian immunodeficiency virus (SIV), feline immunodeficiency virus (FIV), maedi-visna virus, equine infectious anemia virus (EIAV), caraparu arthritis encephalitis virus (CAEV), and such (referred to as lentiviruses in this invention). HIV-1 includes all the major (M) subtypes (including A to J), N, and outlier (O) (Hu, D. J. et al., JAMA 1996, 275: 210-216; Zhu, T. et al., Nature 1998, 5: 391(6667): 594-7; Simon, F. et al., Nat. Med. 1998, 4(9): 1032-7). Examples of isolated strains of SIV are SIVagm, SIVcpz, SIVmac, SIVmnd, SIVsnm, and SIVsyk. Retroviruses derived from a Spuma virus can also be used, including foamy viruses (DE 4318387; WO 9607749; Virology (1995) 210, 1, 167-178; J. Virol. (1996) 70, 1, 217-22). Viral vectors derived from foamy viruses can be utilized in the introduction of foreign genes into human cells, particularly in gene therapy, for the administration of recombinant vaccines and such.

In addition to the minus-strand RNA virus envelope protein with hemagglutinating activity, pseudotyped viral vectors can further comprise, for example, other envelope proteins of the virus. For example, this invention comprises compositions comprising a pseudotyped viral vector comprising paramyxoviral HN and F proteins, in which a compound is attached to the HN protein. A viral vector comprising a minus-strand RNA virus envelope protein with hemagglutinating activity can further comprise an envelope protein derived from another virus. As such a protein, for example, an envelope protein derived from a virus that can infect human cells is preferable. Such proteins are not particularly limited, and comprise, for example, the amphotropic envelope protein of retroviruses, G protein of vesicular stomatitis viruses (VSV), and such. Furthermore, examples of *Herpesviridae* proteins are gB, gD, gH, and gp85 proteins of herpes simplex virus; and gp350 and gp220 proteins of EB virus. Examples of proteins of *Hepadonaviridae* are S protein of hepatitis B virus. As a retroviral amphotropic envelope protein, for example, an envelope protein derived from murine leukemia virus (MuLV) 4070A strain can be used. Furthermore, the envelope protein derived from MuMLV 10A1 can also be used (e.g. pCL-10A1 (Imgenex), Naviaux, R. K. et al., J. Virol. 70: 5701-5705 (1996)). As an ecotropic envelope protein, for example, an envelope protein derived from Molony's murine leukemia virus (MoMuLV) can be used. As a vesicular stomatitis virus G protein (VSV-G), for example, a protein derived from an Indiana serotype strain thereof (J. Virology 39: 519-528 (1981)) can be used. Besides these, proteins derived from any preferable strains can also be used.

In this invention, minus-strand RNA viruses are viruses comprising a minus-strand (an antisense strand corresponding to the sense strand encoding the viral genes) RNA as the genome, and envelope proteins comprising hemagglutinating activity. Minus-strand RNAs are also referred to as negative strands. Examples of minus-strand RNA viruses are *Paramyxoviridae* Sendai viruses, Newcastle disease viruses, mumps viruses, and measles viruses; *Orthomyxoviridae* influenza viruses; and *Rhabdoviridae* vesicular stomatitis viruses and rabies viruses.

In this invention, viral envelope proteins with hemagglutinating activity are preferably the envelope proteins of single-stranded minus-strand RNA viruses. Single-stranded minus-strand RNA viruses are also referred to as non-segmented minus-strand RNA viruses, comprising viruses belonging to *Paramyxoviridae* (including paramyxoviruses, morbilliviruses, rubulaviruses, pneumoviruses, and such); *Rhabdoviridae* (including vesiculoviruses, lyssaviruses, ephemeroviruses, and such); *Firoviridae; Orthomyxoviridae* (including infuluenza viruses A, B, and C, thogoto-like viruses, and such); Bunyaviridae (including bunyaviruses, hantaviruses, nairoviruses, phleboviruses, and such) and viruses of *Arenaviridae.* Specific examples of envelope proteins with hemagglutinating activity are HN protein of *Paramyxoviridae,* HA protein of *Orthomyxoviridae,* HA protein of influenza virus, G1 protein of *Bunyaviridae,* G protein of *Rhabdoviridae,* VP1 protein of *Firoviridae,* and such.

In this invention, viral envelope proteins with hemagglutinating activity are more preferably derived from paramyxoviruses. "Paramyxoviruses" refer to viruses belonging to *Paramyxoviridae.* Paramyxoviruses includes, for example, paramyxoviruses, morbilliviruses, rubulaviruses, pneumoviruses, and metapneumoviruses; specific examples being Sendai virus, Newcastle disease virus, mumps virus, measles virus, respiratory syncytial virus, rinderpest virus, distemper virus, simian parainfluenza virus (SV5), human parainfluenza virus types 1, 2, and 3, and such. A specific example of a paramyxovirus envelope protein with hemagglutinating activity is HN protein (also referred to as H protein). In this invention, viral envelope proteins with hemagglutinating activity are more preferably those of viruses belonging to a *Paramyxoviridae* subfamily, including paramyxoviruses, morbilliviruses, and rubella viruses, and further more preferably paramyxovirus viral envelope proteins. The viral envelope proteins with hemagglutinating activity are most preferably the Sendai virus HN protein. These viruses can be derived from wild strains, mutant strains, laboratory-passaged strains, and artificially constituted strains. For example, HN protein derived from Sendai virus Z strain can be preferably used. Nucleotide sequences of Sendai virus HN gene are registered at GenBank accession Nos. D26475, M12397, M30202, M30203, M30204, M69046, X00586, X02808, X56131, etc.

Furthermore, as an envelope protein with viral hemagglutinating activity, HA proteins of viruses belonging to *Orthomyxoviridae* are also preferably used. For example, pseudotyped viruses produced using an envelope protein expression plasmid of the influenza virus can infect a wide range of mammalian cells, including human cells. An envelope protein of influenza virus derived from a preferable isolated strain can be used. In the budding of influenza viruses, neuraminidase plays a role in cleaving the bond between HA protein and sialic acid. Therefore, in preparing HA pseudotyped viruses, infective viruses can be obtained by treating the viruses with neuraminidase before, while, or after attaching a compound to HA. Alternatively, by preparing a viral vector that coexists with a protein having neuraminidase activity, the bond with sialic acid can be automatically cleaved. In this case, a viral envelope protein with neuraminidase activity, such as HN protein of paramyxovirus in particular, is preferably used. Thus, the present invention provides compositions with reduced viral hemagglutinating activity comprising a pseudotyped viral vector containing both HA protein of an *Orthmyxoviridae* virus and HN protein of a *Paramyxoviridae* virus, in which a compound is attached to either one or both of the proteins.

The viral envelope proteins may be intact proteins derived from wild-type viruses, or naturally or artificially mutated proteins. For example, it is possible to analyze antigen-presenting epitopes or the like as potential cell surface antigen molecules in these envelope proteins, and then use these epitopes to construct viruses using proteins with lowered antigen-presenting ability. Furthermore, it is also possible to use envelope proteins and such derived from attenuated strains of pathogenic viruses.

For example, using viral envelope proteins with hemagglutinating activity and other envelope proteins whose cytoplasmic domains have been modified by deletion, substitution, and/or addition of amino acid residues, vectors with higher gene-introducing ability can be constructed. The present invention relates to compositions comprising viral vectors that comprise a naturally-occurring viral envelope protein with hemagglutinating activity whose partial or whole cytoplasmic domain is modified by substitution, deletion, and/or addition of amino acid residues, in which a compound is attached to the viral envelope protein with hemagglutinating activity. Specifically, for example, modified proteins obtained by deleting the cytoplasmic domain of HN protein of paramyxovirus, or by substitution or addition in the cytoplasmic domain of other membrane proteins (such as envelope proteins of retroviruses including lentiviruses) are preferably used for producing pseudotyped retroviral vectors with high infection efficiencies.

In this invention, hemagglutinating activity means the activity of causing hemagglutination (HA), that is, the activity of agglutinating erythrocytes. Hemagglutinating activity can be examined by methods known in the art (Kokuritsu Yobou Eisei Kenkyujo Gakuyukai, ed. Second edition, "Virus Experiments", Introduction, pp. 214-225, Maruzen Co., Ltd.). For example, erythrocytes derived from chickens (including chicks and adult chickens), geese, rats, guinea pigs, rhesus monkeys, green monkeys, or humans are available. Reactions are performed under suitable conditions, depending on the proteins, such as at 0°C, 4°C, room temperature, or 37°C. Specifically, hemagglutinating activity can be measured, for example, by the "end-point dilution method" (Kato, A. et al., 1996, Genes Cells 1: 569-579; Yonemitsu, Y. and Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999).

Compounds are attached to the minus-strand RNA virus envelope proteins with hemagglutinating activity in the compositions of this invention, wherein hemagglutinating activity is reduced compared to when the compound is not attached. In this invention, the term "reduced hemagglutinating activity" means significantly reduced hemagglutinating activity, and comprises, for example, activity that is reduced statistically significantly (e.g. a significance level of 5% or more), and also, completely eliminated activity (or activity below the detectable level). The hemagglutinating activity of the compositions of this invention is preferably reduced to 1/2 or less of that of a control composition to which the compound is not attached, more preferably to 1/3 or less, more preferably to 1/5 or less, more preferably to 1/10 or less, more preferably to 1/20 or less, more preferably to 1/50 or less, more preferably to 1/100 or less, more preferably to 1/200 or less, more preferably to 1/500 or less, and more preferably to 1/1000 or less. In contrast, the ability of the compositions of this invention to introduce a pharmaceutical or gene into cells is maintained at a high level compared to the decrease in hemagglutinating activity. That is, when compared to a control composition to which a compound is not attached, the ability of a composition of this invention to introduce a pharmaceutical or gene into a cell is reduced by a smaller degree, or, more preferably, not reduced but rather elevated.

Furthermore, the hemolytic activity of the compositions of this invention is also preferably significantly reduced. Hemolytic activity can be detected by attaching a test composition to blood; leaving the mixture to stand; centrifuging to remove blood cell components; and then measuring the absorbance of the eluted hemoglobin (see Examples). The hemolytic activity of the compositions of this invention is reduced preferably to 70% or less of that of a control composition to which a compound is not attached, more preferably to 60% or less, more perferably to 1/2 or less, more preferably to 1/3 or less, more preferably to 1/5 or less, more preferably to 1/10 or less, more preferably to 1/20 or less, and more preferably to 1/50 or less.

The hemagglutinating activity of the compositions of this invention is reduced, and, in a preferred enbodiment, their hemolytic activity is also decreased; however their ability to introduce a pharmaceutical or gene into the cells is retained. For example, compositions comprising a minus-strand RNA virus or the envelope component of the virus can fuse with the cell membrane of target cells, delivering a pharmaceutical or gene into the cells. A cell membrane fusion ability means the ability to incorporate constituent components into a cell after a complex (carrier) comprised in the composition of this invention has adhered to the cell membrane, using the fusion activity of the complex. This activity is due to the function of fusion (F) protein, which causes cell fusion and is comprised in the envelope of minus-strand RNA viruses. The cell membrane comprised in the compositions of this invention preferably comprises F protein of a minus-strand RNA virus, in addition to the envelope protein with hemagglutinating activity to which a compound is attached.

The ability to fuse with cells can be detected by methods such as: (1) using erythrocyte hemolysis as an indicator; (2) incorporating a fluorescent substance into the membrane and using changes in fluorescence intensity due to dilution by membrane flux during fusion; or (3) encapsulating a pharmaceutical into the aqueous core of a liposome as a cell membrane model, and using leakage of the pharmaceutical as an indicator.

Furthermore, when the compositions of this invention are prepared as pseudotyped viruses from other viruses, such as those of Retroviridae as described above, the compositions are still capable of introducing a pharmaceutical or gene comprised within the virus into cells, due to envelope proteins of the respective viruses. An ability to introduce a pharmaceutical or gene into cells can be confirmed by detecting the pharmaceutical or gene. A pharmaceutical can be detected, for example, by detecting the uptake of a labeled pharmaceutical into cells, or by introducing a biologically active substance into cells and examining changes in cellular characteristics caused by that substance. On the other hand, expression of a gene within the cells can be detected at the RNA level or protein level, by methods such as PT-PCR, Northern blotting hybridization, immunoprecipitation, Western blotting, and such. Alternatively, a gene encoding a biologically active protein can be incorporated into cells, and changes in cellular characteristics caused by that protein can be measured.

Furthermore, the stability of the compositions of this invention in blood is preferably significantly elevated. Elevated stability in blood means a smaller degree of decrease in the composition's ability to deliver a pharmaceutical or gene when a composition of the present invention is placed in blood. The smaller the decrease in the delivery capability of the composition, the higher the composition's stability is in blood. Elevation of stability in blood can be detected by incubating a composition of this invention with blood; measuring the post-incubation level of a pharmaceutical or gene delivered by the composition; and comparing the level with that obtained by a control composition to which the compound is not attached (see Examples). Delivery capability may be referred to as the activity of a pharmaceutical in the case of pharmaceuticals, and as the expression level of a gene in the case of genes. Gene expression can be determined, for example, by directly or indirectly detecting the transcription product (mRNA) or translation product (protein). A biologically active protein can be detected by measuring the activity of the protein. The degree of decrease in the ability of a composition of this invention to deliver a pharmaceutical or gene is preferably reduced to 70% or less of that of a control composition to which the compound is not attached, more preferably to 60% or less, more preferably to 1/2 or less, more preferably to 1/3 or less, more preferably to 1/5 or less, more preferably to 1/10 or less. Compared to an unmodified control composition, it is desirable that the capability of a composition of this invention to deliver a pharmaceutical or gene is elevated relative to its hemagglutinating activity.

In this invention, the phrase "a compound is attached to the envelope protein having hemagglutinating activity" means that the protein is bound to the compound. The binding may be a covalent or noncovalent bond. Examples of noncovalent bonds are hydrogen bonds, coordinate bonds, ionic bonds, hydrophobic interactions (hydrophobic bonds), and intermolecular force (van der Waals forces). The protein and the compound may initially exist separately, and the compound may be attached to the protein via bonding. A compound can be attached to the envelope protein having hemagglutinating activity by, for example, forming covalent bonds by cross-linking. The crosslinks can be formed with any preferred functional groups. Functional groups include, for example, hydroxyl groups, amino groups, aldehyde groups, carboxyl groups, thiol groups, silanol groups, amide groups, epoxy groups, succinylimide groups, phenol groups, and such, but are not limited thereto. Examples of covalent bonds are ester bonds, ether bonds, amino bonds, amide bonds, sulfide bonds, imino bonds, disulfide bonds, etc. The protein and the compound are preferably bound with a non-peptide bond. Methods for forming covalent bonds include cyanogen bromide activation methods, acid azide derivative methods, condensing agent methods, diazotization methods, alkylation methods, etc.

The pharmaceutical- or gene-carrier compositions of this invention can be produced by a method comprising the steps of: contacting a compound with a minus-strand RNA virus envelope protein having hemagglutinating activity; and binding the protein with the compound. The compound is bound to the envelope protein at its domain exposed on the viral surface. Therefore, specifically, the step of contacting the compound with the protein is preferably performed by contacting the compound with virions comprising that protein. Thus, the compound can be specifically attached to a protein involved with the hemagglutinating activity by a domain that is exposed on the viral surface. In particular, this invention reveals that even in the presence of other envelope proteins, the compound will preferentially attach to the envelope protein with hemagglutinating activity on the viral surface via compound crosslinking, significantly reducing hemagglutinating activity. Therefore, it is possible to easily produce a virus in which a compound is attached to the envelope protein having hemagglutinating activity, for example, by the steps of: attaching a compound produced by usual methods to a virus to which a compound is not attached; and then binding the compound to the virus.

Compounds to be attached to the envelope protein having hemagglutinating activity are not limited, as long as they can significantly inhibit the hemagglutinating activity of the protein. It is preferable that these compounds have no significant biological toxicity, and are chemically stable after the protein attachment. Also, the compounds are non-antigenic, or have low antigenicity. Furthermore, the compounds to be attached are preferably water-soluble. In order to effectively inhibit the hemagglutinating activity, the molecular weights of the compounds are preferably 1,600 Daltons (Da) or higher, more preferably 1,800 or higher, more preferably 1,900 or higher, and more preferably 2,000 or higher. Particularly preferable compounds have a molecular weight of 2,800 Da or higher, more preferably 3,800 or higher, more preferably 4,300 or higher, more preferably 4,500 or higher, and more preferably 5,000 or higher. In particular, compounds with a molecular weight of 12,000 Da or higher, more preferably 14,000 or higher, more preferably 15,000 or higher, more preferably 16,000 or hihger, more preferably 18,000 or higher, and more preferably 20,000 or higher can remarkably inhibit hemagglutinating activity.

The compounds to be attached may be various polymers such as organic compound polymers. They may also have an uneven molecular weight distribution, in which case the phrase "molecular weight of a compound to be attached" used herein means an average molecular weight. The distribution of molecular weight (MW/Mn) value is preferably small, for example, 5 or less, preferably 3 or less, more preferably 2 or less, more preferably 1.6 or less, and more preferably 1.4 or less.

Specifically, the natural polymers can be, for example, proteins such as antibodies, albumin, collagen, gelatin, fibrin, lectin, and heparin, or fragments thereof; polysaccharides such as dextran, cyclodextrin, dextran sulfate, cellulose, chitin, chitosan, and alginic acid; and lipids. As the synthetic polymers, polylactic acid, polylysine, polystyrene, pyran polymer, polyglutamic acid, polyacrylamide, polyvinyl alcohol, polyethyleneglycol, styrene-maleic acid copolymer, lysine-glutamic acid copolymer, polyhydroxyethyl methacrylate, Ficoll, and such can be used.

The compound to be attached is exemplified by a polyethyleneglycol (PEG) in particular (J.M. Harris, "Polyethylene Glycol Chemistry. Biotechnical and Biomedical Applications", Plenum, New York, NY, 1992; J.M. Harris and S. Zalipsky, "Chemistry and Biological Applications of Polyethylene Glycol", ACS Books, Washington, D.C., 1997). PEGs are preferably used because of their water solubility, and low or non-toxicity. "PEG" represents compounds comprising the following structure:

$$-(CH_2CH_2O)_n- \hspace{3cm} \text{[Compound 1]}$$

wherein, n is a natural number of 2 or more.

For example, a typical PEG is exemplified by compounds comprising the following structure:

$$HO-(CH_2CH_2O)_n-H \hspace{3cm} \text{[Compound 2]}$$

wherein, n is a natural number of 2 or more.

PEGs can comprise any desired modified derivatives, for example, in which a hydroxyl group or such is substituted in the terminal of the above structure. For example, monomethylether (mPEG), in which a hydroxyl group on one end of the main chain has been substituted, is preferably used in this invention. PEGs may be linear or branched. For example, PEGs comprising one, two, three, or more side chains can be used. These side chains may comprise the ethyleneglycol structure. PEGs of various molecular weights are available. For example, PEG2000 (average molecular weight: 2000), PEG3000 (average molecular weight: 3000), PEG4000 (average molecular weight: 4000), PEG5000 (average molecular weight: 5000), PEG8000 (average molecular weight: 8000), PEG10000 (average molecular weight: 10000), or PEG20000 (average molecular weight: 20000) can be preferably used. By using a PEG that comprises a functional group for cross-linkage, the PEG can be attached to the envelope protein having the hemagglutinating activity.

For example, a PEG comprising a primary amino group on its terminus can be cross-linked with an acylation reagent (Buckmann, A. et al (1981) Makromol. Chem. 182:1379; Zalipsky, S. et al (1983) Eur. Polym. J. 19: 1177; Eidelman, O. et al (1991) Am. J. Physiol. 260 (Cell Physiol 29): C1094; Pillai, V.N.R. et al (1980) J. Org. Chem. 45: 5364). Alternatively, PEG can be simply cross-linked using an N-hydroxysuccinimide (NHS)-activated ester, activated carboxylic acid, activated aldehyde, or such. Specific examples of PEG·carboxylic acid NHS esters are PEG-succinimidyl succinate, PEG-succinimidyl carbonate, PEG-succinimidyl propionate, and PEG-succinimidyl butanoate (Olson, K. et al, (1997) J. M. Harris & S. Zalipsky Eds., Poly(ethylene glycol), Chemistry & Biological Applications, pp 170-181, ACS, Washington, DC.; Harris, J. M. and Kozlowski, A., US Patent No. 5,672,662). Furthermore, benzotriazole carbonate derivatives of PEG form a stable urethane bond with protein amino groups (Dolence, Eric K., US Patent No. 5,650,234). A PEG comprising an aldehyde group on its terminus can be also used. Aldehyde groups can react with amines under milder conditions than for NHS ester. For example, PEG-propionaldehyde can be preferably used (Harris, J.M. et al (1984) J. Polym. Sci. Polym. Chem. Ed. 22: 341; Kinstler, Olaf B. et al., U. S. Patent 5,824,784; Wirth, P. et al (1991) Bioorg. Chem. 19: 133). Furthermore, a maleimide derivative of PEG can be cross-linked to a thiol group (Goodson, R.J. & Katre, N.V. (1990) Bio/Technology 8: 343; Kogan, T.P. (1992) Synthetic Comm. 22, 2417). Carboxymethyl-NHS derivatives, norleucine-NHS derivatives, tresylate derivatives, epoxide derivatives, carbonylimidazole derivatives, and PNP carbonate derivatives of PEG can be also used. In particular, PEG tresylate and PEG succinimidyl propionate can be preferably used.

The compound can be bound, for example, by preparing virions comprising an envelope protein with hemagglutinating activity at a concentration of about 1 mg protein/ml, and attaching the compound preferred to be attached at about 0.1 to 100 mg (preferably about 1 to 50 mg) to the virions. For example, by reacting SPA-PEG5K (1 to 5 mg) or SPA-PEG20K (about 10 to 20 mg) with the virus (1 mg protein) in a buffer solution (pH8.5), it is possible to significantly reduce the hemagglutinating activity of the virus and yet maintain the ability of the virus to introduce a pharmaceutical or gene into cells. Since the attachment of a compound to the protein may be affected by reaction conditions, such as the amount of the compound used in the reaction, conditions are appropriately adjusted so as to significantly reduce the hamagglutinating activity and maintain the ability to introduce a pharmaceutical or gene into cells.

For example, when attaching a compound to a minus-strand RNA virus, the ratio of the amount of compound to virus, expressed as the molar amount of the compound and the protein weight of the virus, can be set at 1 to 10 µmol/mg protein (compound:virus ratio), more preferably 2 to 8 µmol/mg protein, and further more preferably 3 to 8 µmol/mg protein, for example, about 4 µmol/mg protein.

To avoid loss of the ability to introduce a pharmaceutical or gene into cells, the molecular weight of a compound to be attached is preferably 100,000 Da or less, more preferably 80,000 or less, more preferably 60,000 or less, and more preferably 50,000 or less. Particularly, compounds with a molecular weight of 40,000 Da or less, more preferably 35,000 or less, more preferably 30,000 or less, more preferably 25,000 or less, more preferably 20,000 or less, are preferable to maintain the introducing ability and specifically reduce hemagglutinating activity.

As shown in Example 28, when modifying the hemagglutinin protein, the ability to introduce a pharmaceutical or gene was revealed to be even further enhanced by further modifying the protein with an aliphatic polyamine, such as a polyethyleneimine (PEI). "PEI" stands for compounds comprising the following structure:

$$-(CH_2CH_2NH)_n \hspace{3cm} \text{[Compound 3]}$$

wherein, n is a natural number of 2 or more.

For example, attaching PEI to the PEG-modified derivative of a minus-strand RNA virus can enhance its gene-introducing ability significantly. In this case, the ratio of the amount of PEI to virus, expressed as the weight of the compound and the protein weight of the virus, can be set at 0.001 to 1 (compound/virus ratio), more preferably 0.01 to 0.2, and more preferably 0.01 to 0.1. PEI comprising an average molecular weight of, for example, 25 kDa to 750 kDa can be preferably used, but is not limited thereto.

Furthermore, in this invention, it was found that even when the efficiency of pharmaceutical or gene introduction is reduced or lost upon attachment of a compound to a hemagglutinin protein, this introduction efficiency can be retained by attaching a compound with cell-binding activity to a complex comprising this protein. Thus, it has become possible to prepare the pharmaceutical- or gene-carrier compositions of this invention without limiting the molecular weight of the compound to be attached to the envelope protein. The present invention provides pharmaceutical- or gene-carrier compositions comprising a cell membrane containing a minus-strand RNA virus envelope protein with hemagglutinating activity, wherein: (a) a compound is attached to the protein; (b) hemagglutinating activity is reduced compared to a composition to which the compound is not attached; (c) the composition comprises a complex formed from the protein together with a compound comprising cell-binding activity; and (d) the composition has the ability to introduce a pharmaceutical or gene into target cells. Even when a compound is bound to the envelope protein to inhibit hemagglutinating activity and gene introduction, it is possible to inhibit hemagglutinating activity and yet maintain gene introduction ability by further incorporating a ligand in to the compound. A "ligand" is a compound that binds to a receptor on the cell surface, activating the receptor function. For example, when using an antibody $F(ab')_2$ fragment (MW: about 50 kDa), keeping down the amount of binding antibody fragment can retain a gene-introducing ability of the viral vector relative to the residual hemagglutinating activity. However, even when hemagglutinating activity is completely inhibited, a vector's gene-introducing ability can be dramatically enhanced by binding the antibody with a ligand (folic acid) having cell-binding activity (see Examples).

Forming a complex between a compound having cell-binding activity and an envelope protein with hemagglutinating activity means that the compound and the protein bind to each other directly or indirectly (for example, via another compound), by a covalent or non-covalent bond. Bonds may be either covalent or non-covalent. For example, a composition may comprise a cell membrane comprising an envelope protein having hemagglutinating activity and a compound having cell-binding activity. The compound with cell-binding activity must be exposed on the surface of the complex. For example, when the complex is a virion, the protein and the compound may exist on the viral envelope surface. Alternatively, when the complex is a liposome, the protein and the compound may exist on the surface of the liposome. In order to bind a compound with cell-binding activity to the complex, for example, the compound can be cross-linked to a desired envelope protein comprised in the complex, or preferably to an envelope protein with hemagglutinating activity. Cross-links can be formed using a desired functional group in a similar way to that described above. Alternatively, if the compound is a protein, it can be expressed as a membrane protein in the envelope, or as a fusion protein with an envelope protein comprised in the complex. In a preferred embodiment, a compound with cell-binding activity binds directly to the compound attached to the envelope protein with hemagglutinating activity, through a covalent or non-covalent bond. The compound with cell-binding activity can also be the same compound that is attached to the envelope protein with hemagglutinating activity. The present invention relates to methods for producing pharmaceutical- or gene-carrier compositions which comprise a cell membrane comprising a minus-strand RNA virus envelope protein having reduced hemagglutinating activity, wherein the method comprises the steps of: (a) contacting a compound with the protein; and (b) binding the protein and the compound; and further (i) attaching a compound with cell-binding activity to the compound. Step (i) may be performed before step (a), or simultaneously with step (a) or step (b), or in between step (a) and step (b), or after step (b). Step (i) is preferably performed before step (a). Alternatively, a compound that has already undergone step (i) may be attached to the minus-strand RNA virus envelope protein with hemagglutinating activity.

Any preferred compounds can be used as compounds with cell-binding activity, so long as they are capable of binding to a cell surface, and thereby assisting a composition of this invention to introduce a pharmaceutical or gene into cells. For example, compounds binding to cell surface proteins or cell surface sugar chains can be used as such compounds. Specific examples are viral envelope proteins, natural or synthetic ligands, hormones, or cell adhesion factors. Specifically, folic acid, transferrin, erythropoietin, antibody, lectin, galactose, mannose, and such can be used.

Among them, in this invention, folic acid was proved to be extremely effective in maintaining the pharmaceutical- or gene-introducing ability of the complex comprised in the compositions of this invention. The present invention provides pharmaceutical- or gene-carrier compositions which comprise a cell membrane comprising a minus-strand RNA virus envelope protein with hemagglutinating activity, wherein (a) a compound is attached to the protein; (b) hemagglutinating activity is reduced compared to a composition to which the compound is not attached; (c) the composition comprises a complex formed by the protein and folic acid; and (d) the composition has the ability to introduce a pharmaceutical or gene into target cells. In particular, folic acid preferably binds directly to the compound attached to the protein.

When producing the compositions of this invention using a compound with cell-binding activity, a particularly preferable compound to be attached to the envelope protein with hemagglutinating activity is an antibody that binds to the protein. In a preferred embodiment the compositions of this invention are compositions comprising an envelope protein with hemagglutinating activity bound with an antibody or fragments thereof, wherein the envelope protein forms a complex with the compound with cell-binding activity. That is, the present invention relates to pharmaceutical- or gene-carrier compositions which comprise a cell membrane comprising a minus-strand RNA virus envelope protein with hemagglutinating activity, wherein (a) an antibody or fragment thereof that binds to the protein is bound to the protein; (b) hemagglutinating activity is reduced compared to a composition to which the compound is not attached; (c) the composition comprises a complex formed by the protein and the compound with cell-binding activity; and (d) the composition has the ability to introduce a pharmaceutical or gene into target cells. The compound with cell-binding activity is more preferably bound directly to the antibody or fragments thereof.

In this invention, antibodies include monoclonal antibodies and polyclonal antibodies. They further include antisera obtained by immunizing experimental animals such as rabbits with the proteins of this invention, all classes of polyclonal antibodies and monoclonal antibodies, and further, human antibodies, and humanized antibodies that result from gene recombinations. Polyclonal antibodies are produced by, for example, preparing an envelope protein having hemagglutinating activity, or partial peptides thereof; and immunizing a rabbit, goat, sheep, or such with this protein or peptide as an antigen. As an antigen peptide, the extracellular domain of the envelope protein with hemagglutinating activity, or fragments thereof, can be used. Antigen peptides can be appropriately coupled to other proteins, for example, carrier proteins such as keyhole limpet hemocyanin and albumin for immunization. Monoclonal antibodies can be prepared by using the spleen cells of immunized mice or rats to obtain hybridomas that produce monoclonal antibodies. Antibodies can be prepared by methods known in the art (Harlow, E. and Lane, D. (eds.), Antibodies: A Laboratory Manual, Cold Spring Harbor Laboratory Press, 1988; Shepherd, P. and Dean, C. (eds.), Monoclonal Antibodies: A Practical Approach (Practical Approach Series, 227), Oxford Univ Press, 1999). Polyclonal antibodies can be purified from sera, while monoclonal antibodies can be purified from hybridoma culture supernatants or ascites of animals inoculated with hybridomas, using common biochemical techniques such as ammonium sulfate fractionation, protein G Sepharose column, affinitiy columns with an immobilized antigen, etc. In this invention, an antibody that binds to an exterior region (an extracellular domain) of an envelope protein with hemagglutinating activity, is preferably a monoclonal antibody, and more preferably an HN-1 described in Example 12, can be used (Miura et al., Exp. Cell Res., 1982, 141, 409-420). The antibodies in this invention may be antibody fragments, so long as they bind to the envelope proteins with hemagglutinating activity (antigen proteins), and comprise, for example, a Fab, Fab(t), Fab', $F(ab')_2$, antibody variable region fragment (Fv), single chain Fv (scFv), or modified derivative thereof. Fab is a complex consisting of one polypeptide chain comprising an antibody H chain variable region and one polypeptide chain comprising an L chain variable region. These polypeptides bind with each other to form one antigen-binding site (monovalent). Although Fab is typically obtained by digesting immunoglobulins with papain, in this invention, fragments comprising a structure equivalent thereto are also referred to as Fab. Specifically, Fab includes a dimeric protein in which immunoglobulin L chain and polypeptide chains comprising an H chain variable region ($V_h$) and $C_H1$ are linked. Fab' (obtained by digesting immunoglobulin with pepsin followed by cleaving the disulfide bond between H chains), Fab(t) (obtained by digesting immunoglobulin with trypsin), and such also have structures equivalent to that of Fab. Furthermore, F(ab')$_2$ means an antibody whose constant region is deleted, or a protein complex comprising a structure equivalent thereto, and specifically refers to a protein complex of two complexes, consisting of one polypeptide chain comprising an antibody H chain variable region and one polypeptide chain comprising an L chain variable region. F(ab')$_2$ is a divalent antibody comprising two antigen-binding sites. F(ab')$_2$ is typically obtained by digesting an antibody with pepsin at around pH 4, and comprises the H chain hinge region. However, F(ab')$_2$ may be digested with other proteases, or cleaved with pharmaceuticals, or artificially designed. scFv stands for a polypeptide in which an antibody H chain variable region and L chain variable region are comprised in a single polypeptide chain. An H chain variable region and an L chain variable region are joined via a spacer of appropriate length, binding with each other to form an antigen-binding site. Such antibody fragments can be prepared from, for example, descriptions in "New Biochemistry Experiments (Shin Seikagaku Jikken Kouza) 12, Japanese Society of Biochemistry, ed. Molecular Immunology III pp 185-195 (Tokyo Kagaku Dojin)" and/or "Current Protocols in Immunology, Volume 1, (John Wiley & Sons, Inc.)". Antibody fragments can be obtained by digesting antibodies with proteolytic enzymes such as pepsin, papain, or trypsin. Alternatively, antibody fragments can be prepared by identifying the amino acid sequences of the variable regions, and expressing these sequences as recombinant proteins. Furthermore, antibodies also include humanized antibodies or human antibodies. Antibodies can be purified by affinity chromatography using a protein A column, protein G column, and such.

In this invention, it is particularly preferable to attach fragments comprising an antibody variable region (antigen-binding region) to the envelope protein with hemagglutinating activity. Any preferred fragments that comprise variable regions of H chain and/or L chain of antibody can be used as such fragments, including, for example, Fab, Fab(t), Fab', F(ab')$_2$, Fv, scFv, or modified derivatives thereof.

In this invention, the pharmaceuticals to be transferred into cells may be any preferred compounds, such as naturally occurring compounds, synthetic compounds, inorganic or organic compounds, and small or macromolecular compounds. Furthermore, the genes to be delivered by a composition of this invention may be any preferred nucleic acids, exemplified by nucleic acids such as DNA or RNA, and derivatives thereof. Nucleic acids may be cyclic and linear single- or double-stranded deoxyribonucleic or ribonucleic acids. Examples of nucleic acid derivatives are the phosphothioate and phosphodithioate. Specific examples of pharmaceuticals or genes to be delivered by the compositions of this invention are: antisense nucleic acids (Drug Delivery System, 10, 91-97, 1995); decoy (Journal of Biological Chemistry, 267, 12403-12406, 1994); ribozymes (Drug Delivery System, 10, 91-97, 1995); triple-stranded DNAs (Saibokogaku, vol. 13, No. 4, 277-285, 1994); plasmid DNAs (Methods Enzymology, 221, 317-327, 1993); RNA vectors; and complexes of these substances and carriers (Proceedings of National Academy of Sciences of United States of America, 89, 7934-7938, 1992) or proteins (Journal of Biological Chemistry, 266 (6), 3361-3364, 1991); and anticancer drugs, antiviral drugs, toxins (diphtheria toxin (Biochim Biophys Acta, 1192, 253-262, 1994); lysine (Biochim Biophys Acta, 1070, 246-252, 1991)), enzymes (Immunology, 81, 280-284, 1994), and other preferred biologically active substances. A preferred gene can also be inserted into a genomic nucleic acid and expressed from a recombinant virus.

The compositions of this invention can be used in direct administration (*in vivo*) and indirect administration (*ex vivo*) to the living body, and furthermore, can be administered into cells outside the living body (*in vitro*). In *in vivo* application, the composition may be administered through any preferred administration route, as long as the pharmaceutical or gene can reach a target cell or tissue. Depending on the properties of the active ingredients, administration can be performed, for example, orally, percutaneously, intranasally, perbronchially, intramuscularly, intraperitoneally, intravenously, intraarticularly, or subcutaneously, but is not limited to these routes. Furthermore, the compositions can be administered systemically or locally. In *in vitro* (including *ex vivo*) administrations, the compositions of this invention are attached so as to contact target cells, for example, they are attached into cell cultures. Since the compositions of this invention are extremely stable in blood in particular, they are useful as injections that are used for administration into the blood, such as for intravenous injections.

Complexes comprising a cell membrane comprising a minus-strand RNA virus envelope protein with hemagglutinating activity, prepared by a method of this invention, can be combined with a preferable and pharmacologically acceptable carrier or vehicle, as necessary. "Pharmacologically acceptable carriers or vehicles" are materials that can be administered together with the complex, and do not significantly inhibit the introduction of a pharmaceutical or gene into cells via this complex. Compounds can be formulated by appropriately combining, for example, sterilized water, physiological saline, culture medium solution, serum, and phosphate-buffered saline (PBS). When a minus-strand RNA vector is proliferated in hen eggs, the composition may contain allantoic fluids. Furthermore, the compositions of this invention may comprise carriers or vehicles such as deionzied water and 5% dextrose aqueous solution; stabilizers for liposome membranes (e.g. sterols such as cholesterol); antioxidants (such as tocopherol and vitamin E); and additionally, plant oils, suspension agents, surfactants, stabilizers, biocides, and such.

Furthermore, preservatives and other additives can be attached thereto. The compositions of this invention may be in any form, such as an aqueous solution, capsule, suspension, or syrup, and also in the form of a solution, freeze-dried preparation, or aerosol. In the case of freeze-dried preparations, the compositions of this invention may comprise, as a stabilizer, sorbitol, sucrose, amino acid, various proteins, etc. Compositions comprising a minus-strand RNA viral vector are useful as reagents and pharmaceuticals.

Those skilled in the art can appropriately determine the dose of the compositions of this invention, which varies depending upon the disorder, patient body weight, age, gender, and symptoms, the purpose of administration, dosage form of composition, administration method, type of pharmaceutical or gene to be introduced, and such. When the composition comprises an infective minus-strand RNA virus, it is preferably administered with a pharmaceutically acceptable carrier, preferably in a dose in the range of about $10^5$ CIU/ml to about $10^{11}$ CIU/ml, more preferably about $10^7$ CIU/ml to about $10^9$ CIU/ml, and most preferably about $1x 10^8$ CIU/ml to about $5x 10^8$ CIU/ml. In humans, a single dose is preferably in the range of $2x 10^9$ CIU to $2x 10^{10}$ CIU, and administration may be carried out once or a number of times within a clinically acceptable range of side effects. The same can be said of the number of daily administrations. For non-human animals, an amount can be administered that is calculated from the above-described doses, based on the body weight ratio or volume ratio of target site of administration (e.g. average values) between a target animal and a human.

The animals to be administered the compositions of this invention are not particularly limited, and are, for example, birds, mammals, and other vertebrates, such as chickens, quails, mice, rats, dogs, pigs, cats, bovine, rabbits, sheep, goats, monkeys, and humans. When the methods of this invention are used *in vitro* (including *ex vivo*), for example, humans, non-human mammals, and birds can be used. When the methods of this invention are used *in vivo,* non-human mammals and birds are used in particular.

Furthermore, the present invention relates to an antibody, or fragments thereof, that bind to a minus-strand RNA virus envelope protein with hemagglutinating activity, wherein a compound binding to the cell is coupled to the antibody or fragments thereof. Such an antibody, or fragments thereof, bind to the envelope protein via attachment to a minus-strand RNA virus or processed products thereof, and thus reduces hemagglutinating activity, while a cell-binding compound maintains the function of assisting in viral adhesion to the cell. Therefore, such an antibody or fragments thereof are extremely useful in producing the pharmaceutical- or gene-carrier compositions of this invention. The antibody or fragments thereof may be any preferred fragments comprising variable regions of an L chain and/or H chain of an antibody as described above. More specifically they are preferably Fab, Fab(t), Fab', $F(ab')_2$, Fv, scFv, and such. Among them, $F(ab')_2$ is preferably used, in particular. As the cell-binding compounds, ligands of cell surface receptors are particularly preferred, such as folic acid transferrin, erythropoietin, antibody, lectin, galactose, mannose, etc.

The antibodies of this invention, or fragments thereof, can be formed into a composition together with pharmaceutically acceptable carriers. Such carriers comprise, for example, water, alcohol, glycerol, salts, proteins, and gelatin, and pH buffering agents, stabilizers, suspending agents, and preservatives known in the art.

Furthermore, this invention relates to kits which comprise: (a) an antibody or fragments thereof binding to a minus-strand RNA virus envelope protein with hemagglutinating activity, wherein a compound is bound to the antibody or fragments thereof; and (b) a pharmaceutical- or gene-carrier composition which comprises a cell membrane comprising the envelope protein. Examples of a pharmaceutical- or gene-carrier composition comprising a cell membrane that comprises the envelope proteins include infective virions, inactivated viruses, or liposomes comprising a viral envelope. Infective virions may be, for example, minus-strand RNA viruses or other viruses comprising an envelope protein with hemagglutinating activity of the minus-strand RNA virus. A pharmaceutical- or gene-carrier composition with reduced hemagglutinating activity can be prepared by adding an above-described antibody or fragments thereof, to these viruses, and thereby binding the antibody or fragments thereof to the envelope protein. For example, hemagglutinating activity can be appropriately controlled, according to the purpose, to enhance stability in blood or such for *in vivo* administration. These compositions can be easily prepared by mixing kit components prior to use, thus producing a very easy to use delivery system.

Brief Description of the Drawings

Fig. 1 is a diagram showing PEG-modification of the envelope proteins (HN and F proteins) of SeV vector, using TPEG- and SPA-PEG modification reagents.

Fig. 2 is a graph of the results of measuring SeV vector and TPEG-modified SeV vector hemolytic activity toward rat blood, at 37°C for 15 minutes.

Fig. 3 is a bar graph showing the results of examining SeV vector and TPEG-modified SeV vector stability in rat blood. After treatment at 37°C for one, 15, and 30 minutes, the plasma was separated and HeLa cells were infected. After 24 hours, the expression level of *LacZ* gene was quantified. The results are shown as the mean value of three determinations ± the standard deviation.

Fig. 4 is a photograph showing the SeV vector and TPEG-modified SeV vector subjected to SDS electrophoresis and then silver staining. Arrows indicate the electrophoretic positions of HN protein and F protein. Lane 0 corresponds to the SeV vector, while lanes 8 to 10 correspond to sample numbers 8 to 10 in Table 2, respectively.

Fig. 5 is a series of photographs showing the results of using X-gal staining method to assess *LacZ* expression levels after introducing the SeV vector and SPA-PEG5K-modified SeV vector into LLCMK2 cells, in the presence of rabbit anti-SeV antiserum (10-, 100-, and 1,000-fold diluted sera).

Fig. 6 is a diagram illustrating the selective modification of HN protein comprised in the SeV vector with a folic acid-bound anti-HN monoclonal antibody.

Fig. 7 is a graphic representation of the HAU of SeV vectors modified with HN-1, HN-1/F(ab')$_2$, or the folic acid-bound antibodies thereof, prepared by NHS method. The y-axis represents antibody concentration, and the x-axis represents the logarithm of hemagglutinating activity (log$_2$(HAU)).

Fig. 8 is a bar graph representing the hemolytic activity of SeV vector, HN-1-modified SeV vector, and folic acid-bound HN-1-modified SeV vector toward chicken erythrocytes. The results shown were obtained for SeV vector at 1x 10$^7$, 1x 10$^8$, and 1x 10$^9$ pfu/ml. Modified vectors are 1x 10$^9$ pfu/ml of each SeV vector modified with 100 µg/ml of antibody. The x-axis in the figure is the absorbance at 570 nm, showing the amount of released hemoglobin measured.

Fig. 9 is a bar graph representing *LacZ* gene expression two days after infecting KB cells with SeV vector, HN-1-modified SeV vector, and folic acid-bound HN-1-modified SeV vector. In the infection experiments, conditions of excessive folic acid were created by adding 1 mM folic acid into the culture medium. The results are shown as the mean value of three determinations ± the standard deviation.

Fig. 10 is a bar graph showing *LacZ* gene expression two days after infecting KB cells with SeV vectors modified with HN-1, HN-1/F(ab')$_2$ or their folic acid-bound derivatives. The results are shown as the mean value of three determinations ± standard deviation.

Fig. 11 is a graphic representation of changes in HAU for SeV vectors modified with HN-1 or folic acid-bound HN-1, prepared by the EDC method, in the presence of various amounts of antibodies. The y-axis represents antibody concentration, and the x-axis represents the logarithm of erythrocyte agglutination activity (log$_2$(HAU)).

Fig. 12 is a graphic representation of changes in the PEG-modified SeV titer. SeV was modified with varying amounts of PEG, and the relative infection efficiency (%) of modified SeV vectors was examined.

Fig. 13 is a graphic representation of changes in the HA activity of PEG-modified SeV. SeV was modified with varying amounts of PEG, and the HA activity of modified SeV vectors was examined.

Fig. 14 is a bar graph showing the correlation between the infectivity and HA activity of PEG-modified SeV, indicating that PEG-modification can increase infectivity relative to HA activity.

Fig. 15 is a series of photographs showing the results of analyzing constituent proteins of PEG-modified SeV using silver staining. Bands derived from envelope F and HN proteins decreased as the reacting PEG amount was increased, and alternative bands appeared in macromolecular fractions.

Fig. 16 is a series of photographs showing the results of analyzing constituent proteins of PEG-modified SeV by Western blotting.

Fig. 17 represents a series of bar graphs and photographs showing the resistance of PEG-modified SeV against neutralizing antibody. Resistance was compared under conditions where the total virion number (total protein amount) of modified SeV was fixed.

Fig. 18 illustrates an *in vivo* assessment of PEG10K-modified SeV, showing the administration schedule and administration conditions of modified SeV, and changes in the anti-SeV antibody titer.

Fig. 19 is a series of photographs representing an *in vivo* assessment of PEG10K-modified SeV, showing expression of the introduced gene in a lung, for the *in vivo* administration of the modified SeV of Fig. 18.

Fig. 20 is a series of bar graphs showing the holding effect of PEG-modified SeV infectivity under 4°C storage conditions.

Fig. 21 is a bar graph showing the modification effect of polyethyleneimine (PEI750K). PEI increased the infectivity of PEG-modified SeV.

Best Mode for Carrying Out the Invention

Hereinafter, the present invention will be specifically described using Examples, but is not to be construed as being limited thereto. References cited herein are all incorporated as a part thereof.

[Example 1] Preparation and purification of SeV vector comprising NLS-*LacZ* gene

NLS-*LacZ*/SeV carrying *LacZ* gene with a nuclear localization signal (NLS-*LacZ*) was prepared by a previously published method (Kato et al., Genes Cells, 1996, 1, 569-579; Hasan et al., J. Gen. Virol., 1997, 78, 2813-2820). This vector was inoculated to 10-day-old embryonated hen eggs. After incubation at 35.3°C for three days, allantoic fluids were harvested, and centrifuged at 4,000 rpm for 15 minutes. The obtained supernatant was then centrifuged at 10,000 rpm for one hour to precipitate the vector. After resuspension in PBS, the vector was layered on a sucrose density gradient (30%/50%), and centrifuged at 25,000 rpm for one hour (in a Beckman rotor SW28). The vector at the sucrose interface was harvested, centrifuged, precipitated, and then resuspended in PBS to prepare a stock solution of purified vector (hereinafter, described as SeV vector). The protein concentration of the vector was measured by a BCA protein assay (Pierce), using the vector solubilized with an equal volume of 2% SDS solution as a sample, and using BSA as the standard. Hemagglutination units (HAU) were determined as described below. In a 96-well round bottom plate (Asahi Technoglass), the vector was serially diluted two-fold with PBS (50 $\mu$l per well). 50 $\mu$l of 1% chicken erythrocytes solution washed with PBS was added to each well, incubated at 4°C for one hour, and then assessed. HAU was expressed as the maximum vector dilution ratio that caused hemagglutination. Furthermore, plaque forming units (pfu) were calculated using the following formula: 1 HAU of SeV = about $10^6$ pfu/ml. For purified vector, the formula was as follows: 1 mg of protein/ml = 4,096 to 5,120 HAU/ml = about 5x $10^9$ pfu/ml.

[Example 2] Preparation of TPEG-modified SeV vector

The SeV vector stock solution in Example 1 was diluted to 2 mg protein per ml in PBS. 0.5 ml of 0.5 M borate buffer (pH8.5) was added to 0.5 ml of this solution to prepare a 1 mg protein/ml solution of pH8.5. 1 mg of Tresyl-activated PEG reagent (TPEG, MW: 5,000) (Shearwater Polymers) was added to the above-described solution in small amounts while stirring, and reacted at room temperature for 90 minutes (Fig. 1). After the reaction was completed, the reaction mixture was diluted with ice-cold PBS (14 ml), and the vector was recovered by centrifuging at 15,000 rpm for one hour (in a Beckman rotor SW28.1). The vector was resuspended in PBS (0.8 ml), and the TPEG-modified SeV vector was obtained. Protein concentration and HAU of the modified vector were determined as in Example 1.

[Example 3] *In vitro* gene expression using TPEG-modified SeV vector

The SeV vector stock solution in Example 1 was diluted to 1x $10^5$ pfu/ml in PBS. The protein concentrations of the TPEG-modified SeV vector solution in Example 2 and the unmodified SeV vector solution were normalized, thus rendering an equal number of virions for both vectors. The day before the experiment, HeLa cells (derived from human cervical carcinoma) were plated on a 12-well plate (Sumitomo Bakelite) at 5x $10^4$ cells/well (in 1 ml/well of MEM medium supplemented with 10% inactivated fetal calf serum (FCS)). The medium was reduced to 0.5 ml, 50 $\mu$l of the above-described diluted vector solutions were added to each well (equivalent to 5x $10^3$ pfu/well when converted into SeV vector, moi= 0.1), and infection was carried out at 37°C in the presence of 5% $CO_2$. After one hour, the vector was removed and the cells were washed twice with medium. Fresh medium (2 ml) was added to the cells, which were then further incubated at 37°C in the presence of 5% $CO_2$ for 24 hours. *LacZ* gene expression was measured using the Galacto-Light Plus™ (Tropix) protocol, and cellular proteins were measured using the BCA method (Pierce). The results were expressed in light units (RLU/$\mu$g protein).
Table 1 shows the HAU of TPEG-SeV vector and gene expression level by the vector. For the same protein concentration, that is, for the same number of virions, the HAU of TPEG-SeV vector was 1/2,000 or less of that of SeV vector. At the same time, for the same number of virions, TPEG-SeV vector retained approximately 50% of the gene expression level in HeLa cells. Thus, it has now become possible to prepare a vector in which only HAU is remarkably reduced, without seriously damaging its ability to introduce a gene into cells.

Table 1

| | Protein Concentration (mg/ml) | HAU/mg | *LacZ* Expression Level (RLU/$\mu$g cell protein) |
|---|---|---|---|
| SeV Vector | 0.93 | 5120 | 2335604 $\pm$ 72774 |
| TPEG-Modified SeV Vector | 0.99 | 2 | 1046353 $\pm$ 29841 |

[Example 4] Hemolytic activity assay

Fresh blood, collected from the carotid artery of SD strain rats under ether anesthetia using a heparinized syringe, was used in experiments. The SeV vector of Example 1 and the TPEG-modified SeV vector of Example 2 were diluted in PBS to prepare a series of solutions comprising 1 to 1000 $\mu$g protein per ml.
A diluted solution of vector (50 $\mu$l) and rat blood (50 $\mu$l) were mixed and incubated for 15 minutes at 37°C. After cooling on ice at 4°C, plasma was separated by centrifugation. The amount of hemoglobin released into the plasma was measured using absorbance at 570 nm (OD570). OD570 when PBS was used in place of the vector solution was used as the negative control. The OD570 of distilled water was used as the positive control (100% hymolysis). The hemolytic activity of the vector was calculated using the following formula:

$$\text{Hemolytic activity (\%)} = [(\text{OD570 of the vector solution}) - (\text{OD570 with PBS})]/[(\text{OD570 of distilled water}) - (\text{OD570 with PBS})] \times 100.$$

As shown in Fig. 2, SeV vector showed strong concentration-dependent hemolytic activity. On the other hand, the hemolytic activity of TPEG-modified SeV vector was found to be extremely low. 1000 µg of TPEG-modified SeV vector had a hemolytic activity equivalent to that of 5 µg of SeV vector, indicating an approximately 200-fold improvement of vector safety by TPEG-modification.

[Example 5] Stability test in rat blood

The SeV vector of Example 1 was diluted with PBS to 1x $10^8$ pfu/ml. The TPEG-modified SeV vector of Example 2 was diluted with PBS so that the viral protein concentration was equal to that of the SeV vector. 250 µl of diluted vector solution and 250 µl of fresh SD rat blood (male) were mixed, and incubated at 37°C. After a predermined time, an 80 µl blood sample was collected, and plasma was recovered by centrifuging at 4°C. Using 50 µl of a solution prepared by diluting 10 µl of plasma 50-fold in PBS, LacZ gene expression in HeLa cells was examined by the method set forth in Example 3.
As shown in Fig. 3, SeV vector was significantly destablized immediately after blood treatment, and the 15-minute blood treatment decreased its gene expression ability to 1/1000 of the untreated SeV vector. On the other hand, the stability of TPEG-modified SeV vector in blood was improved, indicating remarkably high gene expression ability compared to the unmodified vector.

[Example 6] Preparation of SPA-PEG2K-modified SeV vector

The SeV vector stock solution in Example 1 was diluted with PBS to adjust the concentration to 2 mg protein/ml. This solution was diluted two-fold with 0.5 M borate buffer (pH8.5) to prepare a 1 mg/ml protein solution. Succinimidyl propionic acid derivative PEG reagent (molecular weight 2000) (SPA-PEG2K, Shearwater Polymers) (1, 2, or 4 mg) was added to the above-described solution in small amounts while stirring, and this was reacted at room temperature for 30 minutes (Fig. 1). After completing the reaction, the reaction solution was diluted with 14 ml of ice-cold PBS, and centrifuged at 13,000 rpm for one hour (in a Beckman rotor SW28.1). The vector was collected and then resuspended in 0.8 ml of PBS to obtain the SPA-PEG2K-modified SeV vector (Table 2, sample Nos. 1 to 3). Protein concentration of the modified vector was determined as for Example 1.

Table 2

| Sample Name | Sample No. | Reaction Amount of PEG (mg) | HAU/mg Protein | *LacZ* Gene Expression ratio (%) |
|---|---|---|---|---|
| SeV Vector (Example 1) | | - | 4096 | 100.0 ± 7.5 |
| SPA-PEG2K Modified (Example 6) | 1 | 1 | 1024 | 29.1 ± 2.6 |
| | 2 | 2 | 512 | 4.7 ± 0.2 |
| | 3 | 4 | <1 | 0.5 ± 0.01 |
| SPA-PEGSK Modified (Example 7) | 4 | 1 | 2048 | 115.4 ± 14.2 |
| | 5 | 2.5 | 2048 | 93.1 ± 4.2 |
| | 6 | 5 | 512 | 42.7 ± 4.9 |
| | 7 | 10 | <1 | 1.6 ± 0.1 |
| SPA-PEG20K Modified (Example 8) | 8 | 10 | 1024 | 95.3 ± 9.7 |
| | 9 | 20 | 4 | 48.3 ± 1.5 |
| | 10 | 40 | <1 | 0.2 ± 0.02 |

[Example 7] Preparation of SPA-PEGSK-modified SeV vector

SPA-PEG2K in Example 6 was replaced with SPA-PEGSK (MW: 5000) (Shearwater Polymers). The reaction and purification were similarly performed with reaction amounts of 1, 2.5, 5, or 10 mg PEG to obtain SPA-PEG5K-modified SeV (Table 2, sample Nos. 4 to 7).

[Example 8] Preparation of SPA-PEG20K-modified SeV vector

SPA-PEG2K in Example 6 was replaced with SPA-PEG20K (MW: 20,000) (Shearwater Polymers). The reaction and purification were similarly performed with reaction amounts of 10, 20, or 40 mg PEG to obtain SPA-PEG20K-modified SeV (Table 2, sample Nos. 8 to 10).

Fig. 4 shows the results of subjecting SPA-PEG20K-modified SeV vector to SDS-electrophoresis, and then silver staining to stain each constituent protein. As the amount of PEG in reaction increased, the bands derived from HN protein decreased, and instead, bands appeared in higher molecular weight fractions. These bands were thought to be derived from the polymerized PEG-bound HN protein. On the other hand, a small decrease in F protein band was observed. These results showed that, of the two proteins HN and F, the HN protein was mainly modified in the PEG-modification of the SeV envelope.

[Example 9] HAU of SPA-PEG-modified SeV vector and *in vitro* gene expression by the vector

HAUs of SeV vector in Example 1 and PEG-modified SeV vectors in Examples 6, 7, and 8 (sample Nos. 1 to 10) were measured using the method described in Example 1. Furthermore, LacZ gene expression in HeLa cells was measured using the method described in Example 3, except that the MOI was changed to 0.5, and the number of days of incubation was changed to two days.

Table 2 shows HAU and the results of LacZ expression. As the reaction amount of PEG increased, the HAU of the obtained modified SeV vectors decreased. Although LacZ gene expression by PEG-modified SeV vectors decreased compared to that of the SeV vector, by limiting the reaction amount of PEG as shown in Table 2, HAU could be greatly reduced while holding down the decrease in gene expression ability. Thus, by selecting modification conditions, modified vectors with sufficient gene expression ability were found to be obtainable.

[Example 10] *In vitro* gene expression by SPA-PEG-modified SeV vector in the presence of erythrocytes

HeLa cells were prepared as in Example 3. The medium was removed just prior to the experiment, and 300 μl of PBS-washed chicken erythrocytes, adjusted to 1x $10^9$ cells/ml, were added instead. In the control group, PBS was used in place of the erythrocyte solution. 50 μl of each of the SeV vector of Example 1 or the PEG-modified SeV vectors of Table 2 (sample Nos. 1, 2, 4, 5, 6, 8, and 9) were added to the wells to infect the cells at 37°C for a short period of 5 minutes. The amounts of vector added were adjusted based on Table 2, so as to make the expression efficiency the same. As for to Example 3, cells were treated to measure LacZ gene expression levels 48 hours later.

Table 3 shows the gene expression levels of each sample in the presence of erythrocytes. The results were expressed as relative ratios, taking the expression level in the absence of erythrocytes as 100. For the SeV vector, the presence of erythrocytes reduced the level of gene expression to about 20% of that of the control. On the other hand, in PEG-modified samples, a decrease in HAU was accompanied by a reduced interaction of the vector with erythrocytes, indicating a high level of gene introduction efficiency even in the presence of erythrocytes. These results indicate that in a clinical setting, PEG-modified SeV vectors may allow a quick gene introduction mediated by blood.

Table 3

| Sample Name | Sample No. | *LacZ* Gene Expression Ratio in the Presence of Erythrocytes (%) |
|---|---|---|
| SeV Vector (Example 1) | | 22.5 ± 4.2 |
| SPA-PEG2K Modified (Example 6) | 1 | 47.6 ± 2.0 |
| | 2 | 49.2 ± 13.8 |
| SPA-PEG5K Modified (Example 7) | 4 | 34.4 ± 3.4 |
| | 5 | 46.7 ± 4.0 |
| | 6 | 53.9 ± 4.8 |

Table 3   (continued)

| Sample Name | Sample No. | *LacZ* Gene Expression Ratio in the Presence of Erythrocytes (%) |
|---|---|---|
| SPA-PEG20K Modified (Example 8) | 8 | $46.1 \pm 9.7$ |
| | 9 | $49.6 \pm 6.1$ |

[Example 11] Gene introduction by SPA-PEG5K-modified SeV vector in the presence of neutralizing antibody

(1) Preparation of SPA-PEGSK-modified SeV vector

NLS-LacZ/SeV vector was prepared and purified as in Example 1, and a stock solution thereof was prepared. This vector was reacted with 5 mg of SPA-PEG5K, using the method set forth in Example 7, and the SPA-PEG5K-modified SeV vector was prepared. Titers of this vector against LLCMK2 cells were determined as described below. On the day before the experiment, LLCMK2 cells were plated onto a 6-well plate (Sumitomo Bakelite) at $2x\ 10^6$ cells/ well (in 2ml/well of 10% FCS-supplemented MEM medium). The vector was diluted to a concentration of 1 mg protein/ ml in PBS containing 1% FCS, and a 10-fold serial dilution series was further prepared. After removing the medium of the above-described cells, 1 ml of the diluted vector solutions was added to each well, and infection was carried out for one hour at $37°C$ in the presence of 5% $CO_2$. After removing the vector solution, the cells were washed with medium, and 2 ml/well of fresh medium was added. This was further incubated at $37°C$ in the presence of 5% $CO_2$. The medium was removed two days later. After washing with PBS, cells expressing *LacZ* gene were X-gal stained using a $\beta$-Gal staining kit (Invitrogen). The number of infected cells in the visual field of a 200x magnification was counted for any ten random spots in a well. Based on mean cell counts and area of the visual field, the titer in the well as a whole was calculated using the following formula:

$$LacZ \text{ infection titer (CIU/ml) = (number of infected cells) x}$$

$$854.865 \text{ x (dilution ratio)}$$

As a result, titers of SeV vector and SPA-PEGSK-modified SeV vector were $1.4x\ 10^{10}$ CIU/ml and $2.7x\ 10^9$ CIU/ml, respectively.

(2) Gene introduction in the presence of neutralizing antibody

The SeV vector and SPA-PEGSK-modified SeV vector of Example 11-(1) were diluted with PBS to adjust the titer to $1x\ 10^7$ CIU/ml. 10-, 100-, and 1000-fold diluted solutions of anti-SeV antiserum prepared by immunizing rabbits with SeV (treated at $56°C$ for one hour) or PBS (30 $\mu$l each) were added to 30 $\mu$l of the vector solution, and incubated at room temperature for one hour. On the day before experiment, LLCMK2 cells were plated onto a 12-well plate (Sumitomo Bakelite) at $2x\ 10^5$ cells/well (in 1 ml/well of MEM medium supplemented with 10% FCS). The medium was reduced to 0.5 ml, and 40 $\mu$l of the above-described antiserum-treated vector solution was added to each well, to infect cells at $37°C$ in the presence of 5% $CO_2$ for one hour. Then, two days later LacZ gene expression was assessed by X-gal staining, as for Example 11-(1).
As shown in Fig. 5, the gene introduction efficiency of SeV vector was remarkably reduced in the presence of the antiserum. On the other hand, SPA-PEGSK-modified SeV vector could retain sufficient gene introduction ability, even in the presence of the antiserum.

[Example 12] Preparation and purification of anti-HN monoclonal antibody, HN-1

Hybridoma cells that produce the anti-HN monoclonal antibody, HN-1 (Miura et al., Exp. Cell Res., 1982, 141, 409-420), were successively passaged in Hybridoma-SFM medium (Gibco-BRL) at $37°C$ in the presence of 5% $CO_2$ and 1000 ml of a cell suspension comprising $2x\ 10^5$ cells/ml was prepared. The culture was further continued for two weeks, and then the supernatant was obtained by centrifugation at 3,000 rpm for 10 minutes. This supernatant was then filtered through a 0.45 $\mu$m filter. The monoclonal antibody was purified using Protein G column chromatography. The culture supernatant was passed through a 5-ml Protein G column (Pharmacia), equilibrated with 20 mM sodium phosphate buffer (pH7.0), using a peristaltic pump (a P-1 pump: Pharmacia) at a flow rate of 4.2 ml/min, and the antibody was bound to the column. After washing the column with 20 mM sodium phosphate buffer (pH7.0), the antibodies were eluted with 0.1 M glycine-HCl buffer solution (pH2.7). The eluate was neutralized with 1 M Tris-HCl buffer solution (pH9.0), and then concentrated by ultrafiltration (Centriplus-20, molecular weight cutoff 30,000: Amicon). After exchanging the buffer with PBS using a PD-10 desalting column (Pharmacia), which was equilibrated with PBS, the solution was once more condensed by ultrafiltration, and 160 μl of HN-1 PBS solution was obtained. The protein concentration, as determined by BCA protein assay (Pierce) using bovine γ-globulin as a standard, was 37.6 mg/ml.

[Example 13] F(ab')$_2$ fragmentation of the anti-HN monoclonal antibody, HN-1

The purified monoclonal antibody HN-1 of Example 12 was diluted with 0.2 M acetate buffer (pH4.5) to adjust the concentration to 2 mg protein/ml. 2.35 ml of Pepsin solution (Sigma) adjusted to 0.1 mg/ml in 0.2 M acetate buffer (pH4.5) was added to 2.35 ml of the protein solution, and then reacted at 37°C for 21 hours. After neutralizing the reaction mixture with 700 μl of 1 N sodium hydroxide solution, PBS was added to the solution to adjust the total volume to 25 ml. This solution was loaded onto a 1-ml Protein A column (Pharmacia), equilibrated with PBS, and unreacted antibody was absorbed to the column. Fractions that passed through the column were recovered and condensed by ultrafiltration as in Example 12. The condensed solution was further purified by the gel filtration method using a Sephadex 75 column (Pharmacia) equilibrated with PBS. The purified solution was condensed by ultrafiltration to recover a PBS solution (300 μl) of the F(ab')$_2$ fragment of HN-1 (hereinafter abbreviated as HN-1/F(ab')$_2$). Protein concentration was measured as for Example 12, and the obtained HN-1/F(ab')$_2$ was determined to be 4.08 mg/ml.

[Example 14] Preparation of folic acid-bound monoclonal antibody by NHS method

(1) Synthesis of NHS-activated folic acid

Synthesis was performed by the method of Robert et al. (Journal of Biological Chemistry: Feb. 4, 269 (5), 3198-3204, 1994). Specifically, folic acid (Sigma) was dissolved in dimethyl sulfoxide (DMSO) to prepare a 50 mg/ml solution. 250 μl of triethylamine (Pierce), 470 mg of dicyclohexylcarbodiimide (Tokyo Kasei Kogyo Co., Ltd.), and 260 mg of N-hydroxysuccinimide) (NHS) (Sigma) were added in sequence to this solution. The resulting mixture was stirred overnight at room temperature in the dark. The precipitated side products were removed by filtration, and the filtrate was added in drops to 250ml of diethylether while stirring. The yellow precipitate was repeatedly washed with ether, and then dried under reduced pressure to obtain NHS-activated folic acid.

(2) Preparation of folic acid-bound HN-1

100 μl of a PBS solution containing 2.0 mg/ml of the HN-1 solution purified by the method of Example 12, and 100 μl of 0.5 M borate buffer (pH8.0), were mixed and used as a reaction solution. 2.5 μl of the DMSO solution of Example 14-(1) containing NHS-activated folic acid (5 mg or 10 mg/ml) was added drop-wise to the reaction solution. This was then reacted at room temperature in the dark for one hour. The unreacted folic acid was removed using a PD-10 column equilibrated with PBS. The protein fraction was recovered, condensed by ultrafiltration to obtain the folic acid-bound HN-1, and then protein concentration was measured according to Example 12.

(3) Preparation of folic acid-bound HN-1/F(ab')$_2$

*HN*-1 was replaced with an HN-1/F(ab')$_2$ solution, and the concentration of NHS-activated folic acid was changed to 10 mg/ml, and then the reaction and purification were carried out as for Example 14-(2), to obtain folic acid-bound HN-1/F(ab')$_2$.

[Example 15] HA activity of folic acid-bound monoclonal antibody-modified SeV vector

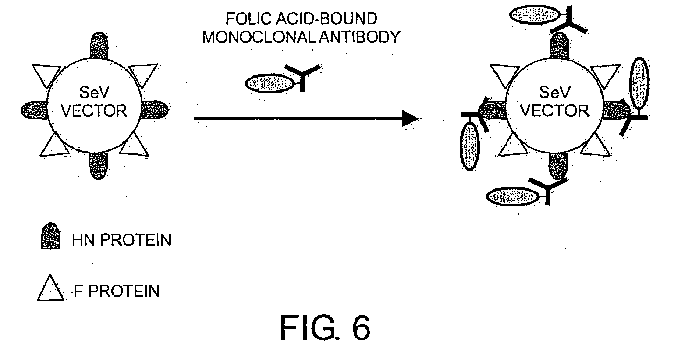

The NLS-LacZ/SeV of Example 1 was diluted with PBS to 1x $10^8$ pfu/ml, and was dispensed into a 96-well round bottom plate at 50 μl per well. The HN-1 purified by the method of Example 12; the HN-1/F(ab')$_2$ of Example 13; and the folic acid-bound HN-1 and folic acid-bound HN-1/F(ab')$_2$ of Example 14 were diluted with PBS to adjust their concentrations to 1 ~ 100 μg protein/ml. 50 μl of the diluted antibody solutions were respectively mixed with the vectors, and left standing at room temperature for 30 minutes to prepare modified vectors (Fig. 6). HAU was then measured by the method of Example 1.

Fig. 7 shows the results of the HAU measurements. In all of the modified SeV vectors, HAU decreased with the increase in the amount of added antibodies, becoming undetectable when the antibody concentration was 10 μg protein/ml or higher. From these results, it became possible to prepare a folic acid-bound antibody-modified SeV vector with a significantly reduced interaction with erythrocytes.

[Example 16] Hemolytic activity of folic acid-bound monoclonal antibody-modified SeV vector

The NLS-LacZ/SeV of Example 1 was diluted with PBS to $1 \times 10^9$ pfu/ml, and dispensed into a 96-well round bottom plate at 50 $\mu$l per well. The HN-1 purified by the method of Example 12, and the folic acid-bound HN-1 by Example 14, were diluted with PBS to a concentration of 100 $\mu$g protein/ml. Diluted solutions of antibodies or PBS (50 $\mu$l each) were mixed with the vector, and left at standing at room temperature for 30 minutes to prepare modified vectors. Hemolytic activity was examined as before, with the exceptions that the rat blood in the method of Example 4 was replaced with $1 \times 10^9$ cells/ml of chicken blood, and the incubation time was changed to 30 minutes.
Fig. 8 shows the results of measuring hemolytic activity. The hemolytic activities of SeV vectors modified with HN-1 and folic acid-bound HN-1 were extremely low, and when compared to SeV vector, hemolytic activity was even lower than the hemolytic activity of SeV vector at 1/100th of its concentration.

[Example 17] An *in vitro* gene introduction experiment for folic acid-bound monoclonal antibody-modified SeV vector

A modified vector was prepared by changing the titer of the SeV vector of Example 15 to $1 \times 10^7$ pfu/ml, and the antibody concentration to 10 $\mu$g/ml. On the day before the experiment, KB cells (derived from human rhinopharyngeal carcinoma) were plated onto a 12-well plate at $2 \times 10^5$ cells/well (in folic acid-free RPMI1640 medium supplemented with 10% FCS: Gibco-BRL, 1 ml/well). One hour before the experiment, the medium was replaced with 0.5 ml of folic acid-free RPMI1640 medium or the same medium supplemented with 1 mM folic acid. 20 $\mu$l of the above-described vector were added to each well, and cells were infected at 37°C in the presence of 5% $CO_2$. Three hours later, the vector was removed, cells were washed twice with the medium, and 2 ml of fresh medium was added to each well. Wells were then incubated for 48 hours at 37°C in the presence of 5% $CO_2$. LacZ gene expression was measured as in Example 3.
Fig. 9 shows the results of *LacZ* gene expression in KB cells. Gene expression by the HN-1-modified SeV vector was 1/100 or less than that by the unmodified SeV vector, indicating a decrease in gene introduction with the loss of HAU. On the other hand, a significant increase in gene expression was observed for the folic acid-bound HN-1 compared to the HN-1 without folic acid, showing about 15% of the gene introduction of the unmodified SeV vector. Gene introduction using the folic acid-bound HN-1 was observed to be inhibited when excessive folic acid was present in the medium. This suggests that gene introduction by the folic acid-bound HN-1 occurs via the binding of folic acid, which is bound to HN-1 as a ligand, to the folic acid receptor of KB cells, followed by fusion mediated by F protein.

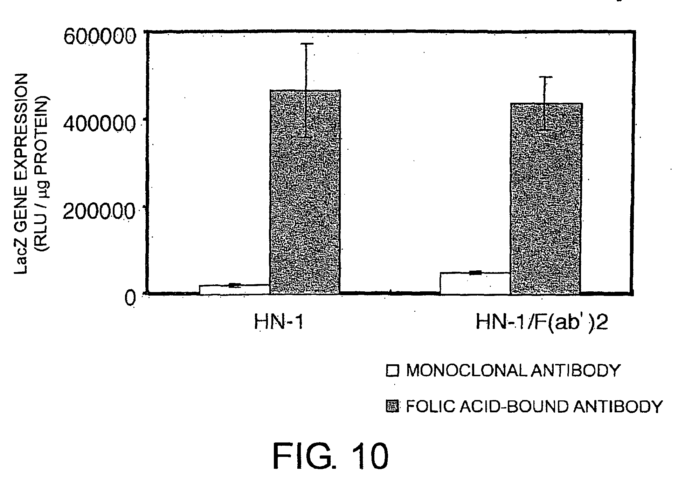

In Fig. 10 a comparison of when the antibody molecule was HN-1/F(ab')$_2$ was carried out. A high level of gene introduction was observed in HN-1/F(ab')$_2$ as well as in HN-1, only when folic acid bound to the antibody.

[Example 18] Preparation of folic acid-bound HN-1 by EDC method and assessment of the modified antibody

(1) Preparation of folic acid-bound HN-1 by EDC method

Preparation was by the method reported by Leamon et al. (Journal of Biological Chemistry, 1992, 267 (35), 24966-24971). That is, 21.7 mg/ml of 1-ethyl-3-(dimethylaminopropyl) carbodiimide (EDC: Sigma) in dimethyl sulfoxide (DMSO) (1 ml) was added to 10 mg/ml folic acid (Sigma) in DMSO (1 ml) . The mixture was then left to stand at room temperature in the dark for 30 minutes. This folic acid-EDC solution (16.6 $\mu$l) was added drop-wise while stirring to the HN-1 solution (300 $\mu$l) of Example 12, adjusted to a concentration of 1 mg protein/ml with PBS. The resulting mixture was reacted at room temperature in the dark for one hour, and then folic acid-bound HN-1 was obtained as in Example 14-(2).

(2) HA activity of folic acid-bound HN-1-modified SeV vector

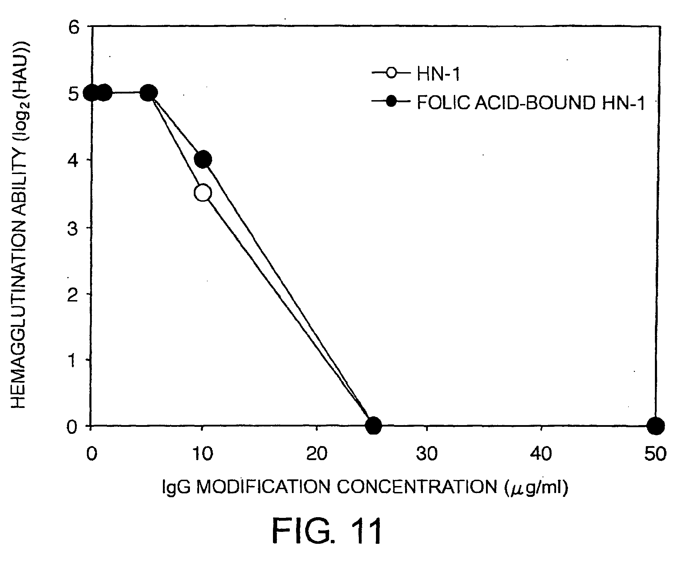

By replacing the folic acid-bound HN-1 of Example 15 with the folic acid-bound HN-1 of Example 18-(1), a modified vector was prepared and HAU was measured.
As shown in Fig. 11, the folic acid-bound HN-1 prepared by the EDC method was also effective in eliminating the HAU by modifying SeV vector.

(3) Gene expression by folic acid-bound HN-1-modified SeV vector

Modified vectors were prepared using a solution of SeV vector ($1 \times 10^7$ pfu/ml) in Example 1, and HN-1 in Example 12 and folic acid-bound HN-1 in Example 18-(1) (both 100 $\mu$g/ml). A549 cells (human lung carcinoma) were used in addition to the KB cells of Example 16, and the infection experiment was similarly carried out.
Table 4 compares the gene expression in KB cells, in which the folic acid receptor is expressed at a high level, and that in A549 cells practically devoid of this receptor (Wang et al., Bioconjugate Chemistry. 1997, 8, 673-679). The folic acid-bound HN-1-modified SeV vector showed a high level gene introduction in KB cells, but only in the absence of excessive folic acid. This indicated that the folic acid ligand that displaces the HN protein hemagglutinating activity mediates the introduction of specific genes by binding to folic acid receptors.

Table 4

| Relative ratio of *LacZ* Gene Expression (where the SeV vector with excessive folic acid (-) is taken as 100%) | | | |
|---|---|---|---|
| | Excessive Folic Acid | KB Cell | A549 Cell |
| SeV Vector | - | $100.0 \pm 10.9$ | $100.0 \pm 6.6$ |
| | + | $107.7 \pm 12.3$ | $142.1 \pm 13.3$ |
| HN-1-Modified SeV Vector | - | $0.5 \pm 0.1$ | $0.8 \pm 0.1$ |
| Folic Acid-Bound HN-1 | - | $14.8 \pm 1.3$ | $3.2 \pm 0.7$ |
| -Modified SeV Vector | + | $2.4 \pm 0.1$ | $2.4 \pm 0.6$ |

[Example 19] Preparation and purification of *GFP*-carrying SeV vector

*SeV/GFP* carrying *GFP* gene was prepared by a known method (Kato et al., Genes Cells, 1996, 1, 569-579; Hasan et al., J. Gen. Virol., 1997, 78, 2813-2820). This vector was inoculated to 10-day-old embryonated hen eggs. After incubating at 35.3°C for three days, allantoic fluids were harvested and centrifuged at 4°C for 30 minutes at 3,000 rpm. The resulting supernatant was centrifuged at 4°C and 35,000 x g for one hour to precipitate the vector. The vector was resuspended in PBS and re-centrifuged at 4°C and 3,000 rpm for 30 minutes, and then the supernatant was centrifuged at 4°C and 35,000 x g for one hour to precipitate the vector. The vector was resuspended in PBS to prepare a stock solution of purified vector (hereinafter referred to as SeV vector). The protein concentration of the vector was measured with a BCA Protein assay (Pierce), using the vector solubilized with an equal volume of 3% Triton X-100 solution as a sample, and BSA as the standard. The concentration of the purified vector was about 1 mg protein/ml.

[Example 20] Preparation of SPA-PEG2K-modified SeV vector

Borate buffer (pH8.5) (0.5 ml) was added to the stock solution of SeV vector in Example 19 (0.5 ml) to prepare a solution of 0.5 mg protein/ml (pH8.5). 0, 2, 4, 6, 8, and 10 mg of succinimidyl derivative of PEG propionic acid reagent (MW: 2000) (SPA-PEG2K, Shearwater Polymers) was added in small amounts to the above-described solution while stirring. This solution was then reacted at room temperature for one hour. After the reaction was completed, the reaction solution was centrifuged at 4°C and 15,000 rpm for one hour. The resulting precipitate was suspended in ice-cold PBS, and re-centrifuged at 4°C and 15,000 rpm for one hour to recover the vector, which was then re-suspended in PBS (0.5 ml) to obtain the SPA-PEG2K-modified SeV vector. The protein concentration of this modified vector was determined as in Example 19.

[Example 21] Preparation of SPA-PEGSK-modified SeV vector

SPA-PEG5K-modified SeV was prepared and purified as in Example 20, after replacing the SPA-PEG2K in Example 20 with SPA-PEG5K of MW: 5000 (Shearwater Polymers), and using PEG reaction amounts of 0, 5, 10, 15, 20, and 25 mg. The protein concentration of this modified vector was determined as in Example 19.

[Example 22] Preparation of PEG2NHS-10K-modified SeV vector

SPA-PEGSK-modified SeV was prepared and purified as in Example 20, after replacing the SPA-PEG2K in Example 20 with PEG2NHS-10K of MW: 10000 (two-branched; Shearwater Polymers), and using PEG reaction amounts of 0, 5, 10, 15, 20, and 25 mg. Protein concentration of the modified vector was determined as in Example 19.

[Example 23] Hemagglutinating activity (HAU) and *in vitro* infectivity (CIU) of PEG-modified SeV vector

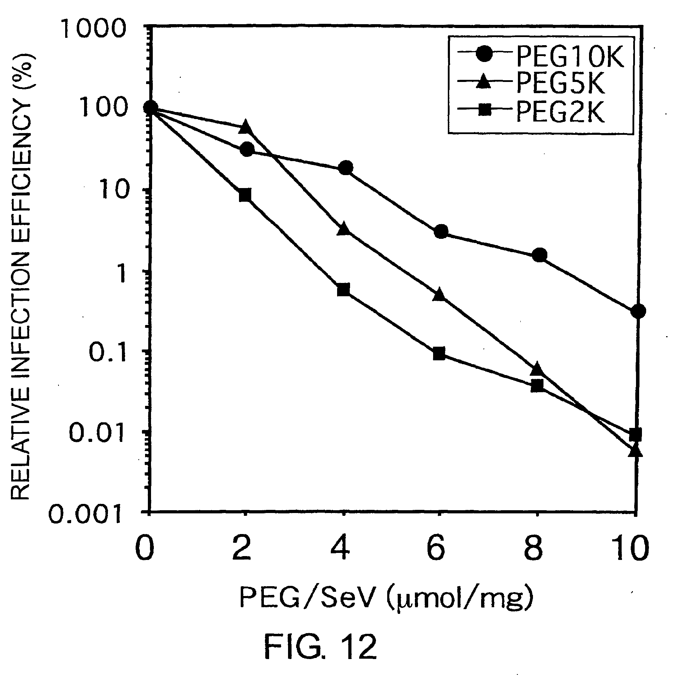

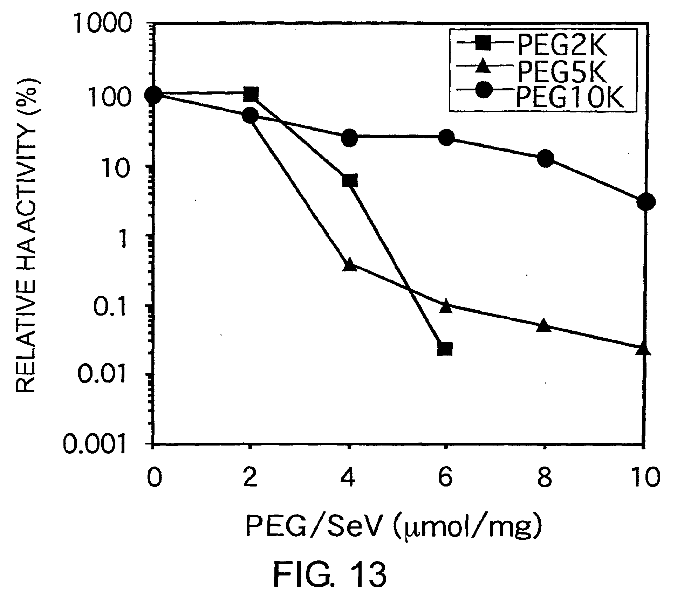

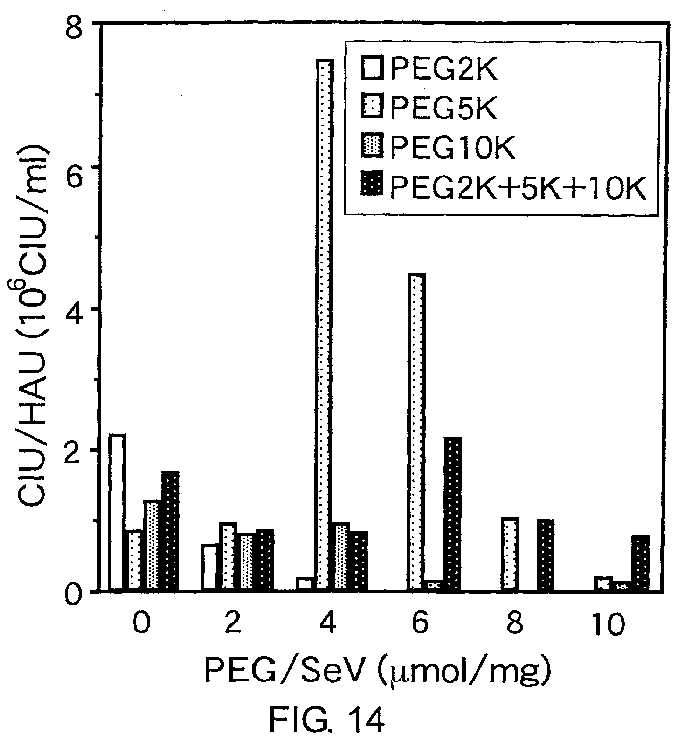

The hemagglutinating activity (HAU) and in vitro infectivity (CIU) of the PEG-modified SeV vectors set forth in Examples 20, 21, and 22 were examined. As shown in Figs. 12, 13, and 14, the HAU and CIU of the above-described three types of PEG-modified SeV vectors were measured, indicating a tendency for reduced HAU and CIU with an increasing amount of PEG in reaction. The larger the molecular weight of PEG derivatives, the smaller the degree of reduction in HAU and CIU caused by increasing their reaction amounts. Furthermore, when the correlation beteen HAU and CIU was examined, the CIU per HAU for SeV modified with an appropriate amount of SPA-PEG5K was about 7-8 times that of the unmodified vector. These results are thought to show the possibility of maintaining infectivity by PEG modification, while reducing hamaggultination and improving the stability of SeV vector in blood.

HAU was determined as follows: Vector was serially diluted 2-fold with PBS (50 µl) using a 96-well round bottom plate (Asahi Technoglass). 50 µl of a 1% solution of chicken erythrocytes, washed with PBS, was added to each well, and then left to stand at 4°C for one hour, whereupon hemagglutination was measured. HAU was expressed as the maximum dilution ratio to cause hemagglutination.

CIU was determined as follows: Vector was serially diluted 10-fold with PBS / 1% BSA, and was then used to infect confluent LLC-MK2 cells in a 24-well plate at 10 µl/well. Two days later, the number of GFP-expressing cells per well was counted to calculate CIU.

[Example 24] Confirmation of PEG modification

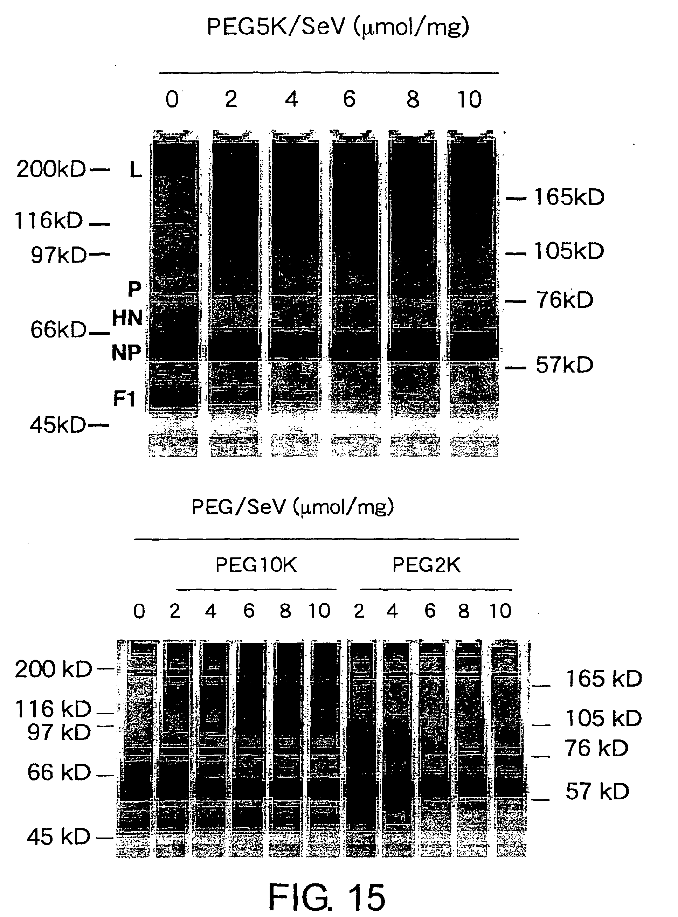

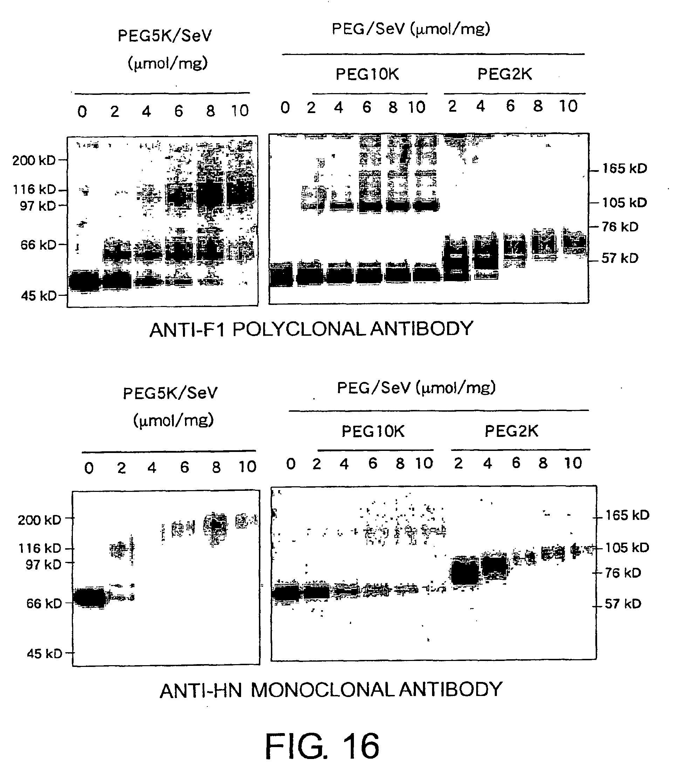

The PEG-modified SeV vectors described in Examples 20, 21, and 22 (0.5 µg/lane) were subjected to SDS-PAGE analysis. As Fig. 15 shows, when the constituent proteins of viral vectors were analyzed by gel silver staining, the bands derived from envelope proteins F and HN decreased with increasing reaction amounts of PEG, and instead, bands appeared in higher molecular weight fractions. A smaller decrease in the amount of F protein compared to HN protein is likely to indicate that more HN proteins have been modified than F proteins. Furthermore, as Fig. 16 shows, when the F and HN proteins, the envelope proteins of the PEG-modified SeV vectors, were analyzed by Western blotting using rabbit anti-F1 polyclonal antibody and mouse anti-HN monoclonal antibody, the obtained results were similar to those obtained by silver staining analysis.

[Example 25] *In vitro* gene introduction by PEG2-NHS10K-modified SeV vector in the presence of neutralizing antibody

In line with Example 22, PEG2-NHS10K-modified SeV was obtained by reaction and purification, using reaction amounts of 0, 15, 20, and 25 mg of PEG. Protein concentration of the modified vector was determined as in Example 19. The vector solution was diluted with PBS / 1% BSA to a concentration of 0.01 µg/µl. The anti-SeV antiserum, prepared by immunizing rabbits (at 56°C for one hour) with SeV, was diluted 10-, 100-, and 1000-fold, and this or 35 µl of PBS / 1% BSA was added to 35 µl of the vector solution, and then reacted at room temperature for one hour. 20 µl of the above-described vector solution, which was treated with the antiserum, was added to each well of a 24-well plate of confluent LLC-MK2 cells, and infection was carried out. GFP expression on the third day after the infection was assessed by taking photographs using a fluorescence microscope, and a fluorescence plate reader was used to quantitatively assess GFP fluorescence intensity at fluorescence wavelength 530 nm and excitation wavelength 485 nm.

As shown in Fig. 17, the unmodified SeV vector showed a tendency for reduced infection efficiency with increasing antibody concentration, however, this decrease was suppressed by modifying the vector with PEG2-NHS10K. The infection efficiency was higher than that of the unmodified vector at all tested antibody concentrations, showing a maximum improvement in infection efficiency of about 70%.

[Example 26] *In vivo* gene introduction by PEG2-NHS10K-modified SeV vector

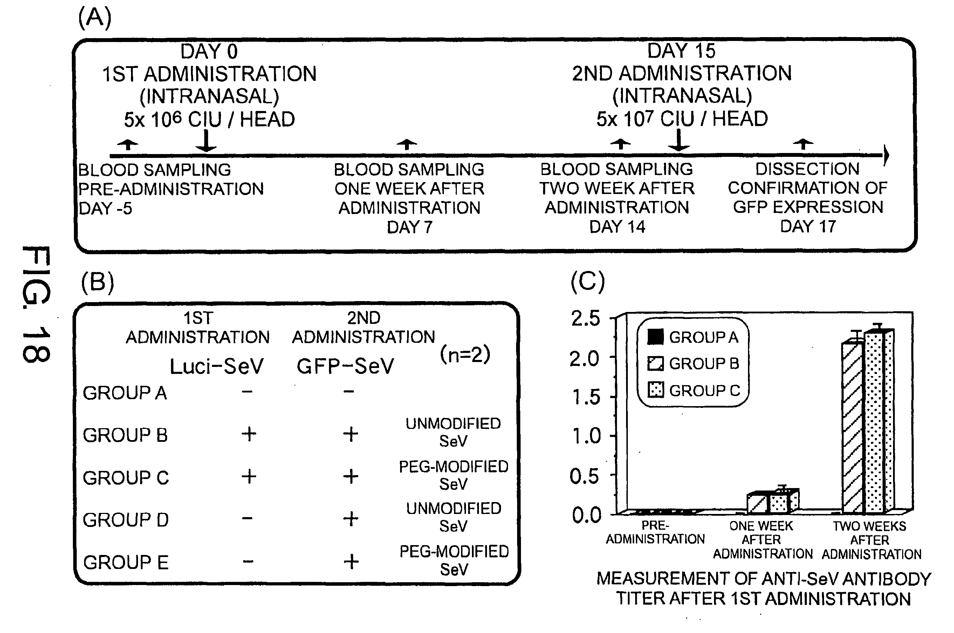

Mice were repeatedly administered with the unmodified SeV control vector prepared with 0mg of PEG, and the PEG-modified SeV vector prepared with a reaction amount of 20 mg of PEG, both prepared in Example 25. As shown in Figs. 18 and 19, mice were divided into five groups; for the first administration, a luciferase-carrying SeV was intranasally administered to mice to produce the anti-SeV antibody; and two weeks later, the second administration was performed using GFP-carrying unmodified SeV as a control in group B and PEG-modified SeV in group C, respectively. Group A served as the untreated control, and groups D and E as the single administration controls. Blood samples were collected from mice prior to the first administration, one week after the first administration, and two weeks after the first administration to measure anti-SeV antibody titers using a commercially available ELISA kit (Prezyme "Seiken", Denka Seiken Co., Ltd.). In mice of groups B and C which had received the first administration, the elevation of the anti-SeV antibody titer was observed. On the day two weeks after the first administration when the antibody titer was sufficiently elevated, the second administration was performed. Two days later, mice were dissected, and GFP expression in lung was observed using a fluorescence microscope to take photographs.

As shown in the fluorescence photographs of Fig. 19, SeV infection was observed over a wide range, and no great differences between the unmodified and PEG-modified vectors were observed. In the second administration, the unmodified SeV showed almost no GFP expression from the full image, and even in the enlarged image, expression was only sparsely observed in portions of the lung surface. On the other hand, the PEG-modified SeV, although had a lower GFP expression than in the first administration, showed more GFP-expressing cells over a wider range compared to the unmodified SeV. As shown in the specifically enlarged image, colonies of GFP-expressing cells were observed.

[Example 27] Effects of maintaining infectivity of PEG-modified SeV at 4°C storage condition

PEG2NHS-20K-modified SeV and PEG2NHS-40K-modified SeV were prepared and purified in the similar fashion by respectively replacing SPA-PEG2K in Example 20 with PEG2NHS-20K of MW: 20000 (two-branched; Shearwater Polymers), using reaction amounts of 10, 20, and 40 mg of PEG; and replacing with PEG2NHS-40K of MW: 40000 (two-branched; Shearwater Polymers), using reaction amounts of 20, 40, and 80 mg of PEG. Protein concentrations of modified vectors were obtained as in Example 19.

CIUs of the above-described PEG-modified SeVs and the untreated SeV stored at 4°C for 8 and 20 days were measured using the method in Example 5. As shown in Fig. 20, in the untreated SeV control, the titer decreased to 1/3 after two weeks of storage at 4°C, while SeVs modified with appropriate amounts of macromolecular two-branched PEGs retained a maximum infection efficiency of nearly 80%.

[Example 28] Improvement of infection efficiency of PEG-modified SeV by PEI modification

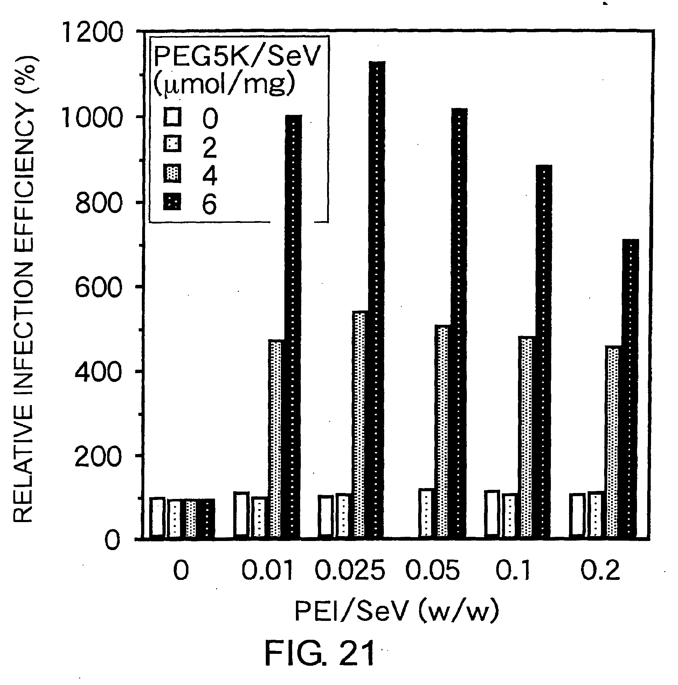

SPA-PEG5K-modified SeV vector prepared with reaction amounts of 0, 5, 10, and 15 mg of PEG in Example 21 was modified using polyethyleneimine of MW: 750000 (PEI; SIGMA), at the PEI:SeV weight ratio of 0, 0.01, 0.025, 0.05, 0.1, and 0.2 to measure the infection efficiency toward LLC-MK2 cells (see Example 23 for the measurement method). As shown in Fig. 21, adding a certain amount of PEI had the effect of increasing the infection efficiency of PEG-modified SeV about 10-fold. The greater the PEG modification, the better the improvement effect PEI has on infection efficiency. Similar results were also obtained using PEI of MW 25000 (Sigma).

Industrial Applicability

An important technical field in pharmacotherapy or gene therapy relates to systems for delivering biologically active substances, such as pharmaceuticals (macromolecular compounds, in particular) and nucleic acids, to target cells or intracellular organelle, namely, drug delivery systems (hereinafter, simply referred to as DDS). DDS can be implemented by using a viral vector, or by administering a composition in which a preferred biologically active substance is enclosed in or carried by an artificial or semi-artificial carrier. The present invention enables the reduction of hemagglutinating activity in both types of DDS, improving stability in blood and gene introduction efficiency. For example, in DDS that use a viral vector, a viral vector with specifically reduced hamagglutinating activity can be produced by applying this invention to a minus-strand RNA viral vector or a pseudotyped viral vector derived from a gene of said virus encoding the envelope protein with hemagglutinating activity, or such. Furthermore, this invention enables the reduction of hemagglutinating activity also in the case of an artificial preparation of liposome and such. Compositions for DDS produced using this invention are extremely effective in *in vivo* application.

**Claims**

1. A pharmaceutical- or gene-carrier composition which comprises a cell membrane comprising a minus-strand RNA virus envelope protein with hemagglutinating activity, wherein:

   (a) a compound is attached to the protein;
   (b) the hemagglutinating activity is reduced compared to a composition to which the compound is not attached; and
   (c) the composition comprises the ability to introduce a pharmaceutical or gene into a target cell.

2. The composition of claim 1, further wherein

   (d) hemolytic activity toward erythrocytes is reduced compared to a composition to which the compound is not attached.

3. The composition of claim 1 or 2, which comprises an infective virion of the minus-strand RNA virus.

4.   The composition of claim 1 or 2, which comprises an inactivated minus-strand RNA virus, or an envelope portion of the virus.

5.   The composition of any one of claims 1 to 4, wherein the minus-strand RNA virus is a *Paramyxoviridae* family virus.

6.   The composition of claim 5, wherein the *Paramyxoviridae* family virus is the Sendai virus.

7.   The composition of any one of claims 1 to 6, wherein the compound attached to the protein has a molecular weight of 1,800 or more.

8.   The composition of claim 7, wherein the compound attached to the protein has a molecular weight of 4,500 or more.

9.   The composition of claim 8, wherein the compound attached to the protein has a molecular weight of 16,000 or more.

10.  The composition of any one of claims 1 to 9, wherein the compound attached to the protein is polyethylene glycol.

11.  The composition of any one of claims 1 to 10, wherein the protein further forms a complex with polyethyleneimine.

12.  The composition of any one of claims 1 to 11, wherein the protein forms a complex with a cell-binding compound.

13.  The composition of claim 12, wherein the cell-binding compound is bound to the compound attached to the protein.

14.  The composition of claim 12 or 13, wherein the compound attached to the protein is an antibody that binds to the protein or fragment thereof.

15.  The composition of any one of claims 12 to 14, wherein the cell-binding compound is a ligand for a receptor on the cell surface.

16.  The composition of claim 15, wherein the ligand is folic acid.

17.  An antibody bound to a cell-binding compound, wherein the antibody binds to a minus-strand RNA virus envelope protein comprising hemagglutinating activity, or a fragment thereof.

18.  The antibody or fragment thereof of claim 17, which is F(ab')$_2$.

19.  The antibody or fragment thereof of claim 17 or 18, wherein the cell-binding compound is a ligand for a receptor on a cell surface.

20.  The antibody or fragment thereof of claim 19, wherein the ligand is folic acid.

21.  A pharmaceutical- or gene-carrier kit which comprises:

   (a) an antibody or fragment thereof which binds to a minus-strand RNA virus envelope protein comprising hemagglutinating activity, and to which a cell-binding compound is bound; and
   (b) a pharmaceutical- or gene-carrier composition comprising the envelope protein.

HN PROTEIN

F PROTEIN

mPEG -O— SO$_2$· CH$_2$CF$_3$
TPEG

OR

mPEG -O— CH$_2$CH$_2$ -C-O -N
SPA-PEG

FIG. 1

FIG. 2

FIG. 3

FIG. 4

SeV VECTOR

SPA-PEG5K MODIFIED
SeV VECTOR

NO SERUM
TREATMENT

1000-FOLD
DILUTED SERUM

100-FOLD
DILUTED SERUM

10-FOLD
DILUTED SERUM

FIG. 5

FIG. 6

FIG. 7

FIG. 8

FIG. 9

FIG. 10

FIG. 11

FIG. 12

FIG. 13

FIG. 14

PEG5K/SeV (μmol/mg)

PEG/SeV (μmol/mg)

FIG. 15

PEG5K/SeV

(µmol/mg)

PEG/SeV (µmol/mg)

PEG10K     PEG2K

0  2  4  6  8  10     0  2  4  6  8  10    2  4  6  8  10

200 kD
116 kD
97 kD
66 kD
45 kD

165 kD
105 kD
76 kD
57 kD

ANTI-F1 POLYCLONAL ANTIBODY

PEG5K/SeV

(µmol/mg)

PEG/SeV (µmol/mg)

PEG10K     PEG2K

0  2  4  6  8  10     0  2  4  6  8  10    2  4  6  8  10

200 kD
116 kD
97 kD
66 kD
45 kD

165 kD
105 kD
76 kD
57 kD

ANTI-HN MONOCLONAL ANTIBODY

FIG. 16

FIG. 17

**FIG. 18**

(A)

DAY 0
1ST ADMINISTRATION
(INTRANASAL)
5x 10⁶ CIU / HEAD

BLOOD SAMPLING
PRE-ADMINISTRATION
DAY -5

BLOOD SAMPLING
ONE WEEK AFTER
ADMINISTRATION
DAY 7

DAY 15
2ND ADMINISTRATION
(INTRANASAL)
5x 10⁷ CIU / HEAD

BLOOD SAMPLING
TWO WEEK AFTER
ADMINISTRATION
DAY 14

DISSECTION
CONFIRMATION OF
GFP EXPRESSION
DAY 17

(B)

| | 1ST ADMINISTRATION Luci–SeV | 2ND ADMINISTRATION GFP–SeV (n=2) |
|---|---|---|
| GROUP A | – | – |
| GROUP B | + | + UNMODIFIED SeV |
| GROUP C | + | + PEG-MODIFIED SeV |
| GROUP D | – | + UNMODIFIED SeV |
| GROUP E | – | + PEG-MODIFIED SeV |

(C)

2.5
2.0
1.5
1.0
0.5
0.0

GROUP A
GROUP B
GROUP C

PRE-ADMINISTRATION

ONE WEEK AFTER ADMINISTRATION

TWO WEEKS AFTER ADMINISTRATION

MEASUREMENT OF ANTI-SeV ANTIBODY
TITER AFTER 1ST ADMINISTRATION

42

GFP EXPRESSION

FIG. 19

A

B

FIG. 20

FIG. 21

## INTERNATIONAL SEARCH REPORT

| | International application No. |
|---|---|
| | PCT/JP03/05527 |

A. CLASSIFICATION OF SUBJECT MATTER
    Int.Cl$^7$ A61K47/48, 35/76, 48/00

According to International Patent Classification (IPC) or to both national classification and IPC

B. FIELDS SEARCHED

Minimum documentation searched (classification system followed by classification symbols)
    Int.Cl$^7$ A61K47/48, 35/76, 48/00

Documentation searched other than minimum documentation to the extent that such documents are included in the fields searched
    Jitsuyo Shinan Koho            1940–1992    Toroku Jitsuyo Shinan Koho    1994–1996
    Kokai Jitsuyo Shinan Koho     1971–1992    Jitsuyo Shinan Toroku Koho    1996–2003

Electronic data base consulted during the international search (name of data base and, where practicable, search terms used)
    CAPLUS(STN), REGISTRY(STN), MEDLINE(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| A | WO 97/16171 A1 (DNAVEC Research Inc.), 09 May, 1997 (09.05.97), Claims 1 to 2; page 11, lines 7 to 25 (Family: none) | 1-16 |
| A | WO 00/27430 A2 (SCHWEIZ, SERUM- & IMPFINSTITUT BERN), 18 May, 2000 (18.05.00), Full text & JP 2002-529427 A | 1-16 |
| A | WO 01/32898 A2 (DNAVEC RESEARCH INC.), 10 May, 2001 (10.05.01), Claims 1 to 2 & JP 2003-513633 A | 1-16 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 July, 2003 (22.07.03) | 05 August, 2003 (05.08.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

International application No.

PCT/JP03/05527

C (Continuation). DOCUMENTS CONSIDERED TO BE RELEVANT

| Category* | Citation of document, with indication, where appropriate, of the relevant passages | Relevant to claim No. |
|---|---|---|
| A | A.G Gitman, Use of Virus-Attached Antibodies or Insulin Molecules To Mediate Fusion between Sandai Virus Envelopes and Neuraminidase-Treated Cells, Biochemistry, 1985, Vol.24, No.11, pages 2762 to 2768 | 17-21 |

Form PCT/ISA/210 (continuation of second sheet) (July 1998)